(19) Europäisches Patentamt / European Patent Office / Office européen des brevets

(11) **EP 4 768 577 A1**

(12) **EUROPEAN PATENT APPLICATION**
published in accordance with Art. 153(4) EPC


(43) Date of publication:
**01.07.2026 Bulletin 2026/27**

(21) Application number: **24855900.7**

(22) Date of filing: **26.08.2024**

(51) International Patent Classification (IPC):
***C12N 9/10*** *(2006.01)* ***C12N 15/63*** *(2006.01)*
***C12P 19/00*** *(2006.01)*

(52) Cooperative Patent Classification (CPC):
**C12N 9/10; C12N 15/63; C12N 15/70; C12N 15/74; C12P 19/00; C12P 19/18; C12P 19/56;**
C12R 2001/19

(86) International application number:
**PCT/CN2024/114579**

(87) International publication number:
**WO 2025/040179 (27.02.2025 Gazette 2025/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **24.08.2023 CN 202311074520**



(71) Applicant: **Abiochem Biotechnology (Group) Co., Ltd.**
**Shanghai 200241 (CN)**

(72) Inventors:
- **WU, Yan**
  **Shanghai 200241 (CN)**
- **ZHENG, Xiaofu**
  **Shanghai 200241 (CN)**
- **XING, Litong**
  **Shanghai 200241 (CN)**
- **TIAN, Zhenhua**
  **Shanghai 200241 (CN)**
- **WANG, Shu**
  **Shanghai 200241 (CN)**
- **XU, Zheng**
  **Shanghai 200241 (CN)**

(74) Representative: **Delbarba, Andrea**
**Bugnion S.p.A.**
**Viale Lancetti, 17**
**20158 Milano (IT)**


Remarks:
The complete document including Reference Table(s) and the Sequence Listing(s) can be downloaded from the EPO website

(54) **PREPARATION METHOD FOR REBAUDIOSIDE M, AND USE OF ß-1,2-GLUCOSYLTRANSFERASE IN SAME**


(57) Provided are a preparation method for rebaudioside D and rebaudioside M, and a use of β-1,2-glucosyltransferase in same. The β-1,2-glucosyltransferase has an amino acid sequence as shown in SEQ ID NO: 4, or has a mutant amino acid sequence having at least 99% sequence identity to the amino acid sequence as shown in SEQ ID NO: 4. The provided β-1,2-glucosyltransferase increases the yield of the intermediate product rebaudioside D when catalyzing rebaudioside A to generate rebaudioside M, thereby correspondingly improving the yield of the final product rebaudioside M.


EP 4 768 577 A1

## Description

This application claims priority of Chinese patent application No. 2023110745202, filed on August 24, 2023, the contents of which are incorporated herein by reference.

### Technical Field

The present disclosure relates to the field of biotechnology, and in particular to the preparation method for Rebaudioside M and application of β-1,2-glucosyltransferase therein.

### Background

Steviol glycosides are sweeteners extracted from stevia (Stevia rebaudiana Bertoni). They have the advantages of high sweetness (e.g., 250 to 450 times sweeter than sucrose), low calories (e.g., only 1/300 of white sugar), cost-effectiveness (only one-third of the price of sucrose), stability (heat-resistant, acid-resistant, alkali-resistant, and not easily decomposed), safety (no toxic side effects), and non-participation in lipid metabolism within the human body. They also have anti-hypertensive, anti-inflammatory, anti-tumor and other effects. Therefore, they have broad applications and high commercial value.

Stevia leaves can accumulate up to 10-20% (on a dry weight basis) of steviol glycosides. The main glycosides found in stevia leaves are rebaudioside A (2-10%), stevioside (2-10%), and steviol glycoside C (1-2%). Other glycosides, such as rebaudioside B, D, E, F, I, M, N and O, as well as steviolbioside and rubusoside, are present in significantly lower amounts in the leaves (approximately 0-0.2%) and can vary depending on the variety of stevia plant and growing conditions.

The structural formula of steviol glycosides is shown in Formula I below, and the specific compounds involved are shown in Table 1 below.

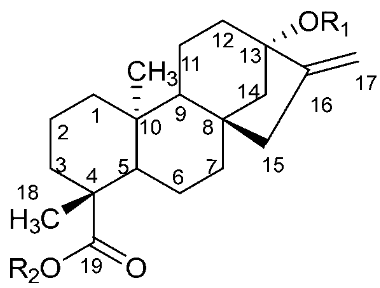

Formula I

Table 1 List of Steviol Glycosides

| Name | | $R_2$ | $R_1$ |
|---|---|---|---|
| 1 | Steviol | H | H |
| 2 | Steviolmonoside | H | β-Glc |
| 3 | Steviolbioside | H | β-Glc-β-Glc(2-1) |
| 4 | Rubusoside | β-Glc | β-Glc |
| 5 | Stevioside (STV) | β-Glc | β-Glc-β-Glc(2-1) |
| 6 | Rebaudioside A | β-Glc | β-Glc-β-Glc(2-1)-β-Glc(3-1) |
| 7 | Rebaudioside B | H | β-Glc-β-Glc(2-1)-β-Glc(3-1) |
| 8 | Rebaudioside C | β-Glc | β-Glc-α-Rha(2-1)-β-Glc(3-1) |
| 9 | Rebaudioside D | β-Glc-β-Glc(2-1) | β-Glc-β-Glc(2-1)-β-Glc(3-1) |
| 10 | Rebaudioside E | β-Glc-β-Glc(2-1) | β-Glc-β-Glc(2-1) |
| 11 | Rebaudioside F | β-Glc | β-Glc-α-Xly(2-1)-β-Glc(3-1) |

(continued)

| Name | | $R_2$ | $R_1$ |
|---|---|---|---|
| 12 | Rebaudioside M | β-Glc-β-Glc(2-1)-β-Glc(3-1) | β-Glc-β-Glc(2-1)-β-Glc(3-1) |
| 13 | Dulcoside A | β-Glc | β-Glc-α-Rha(2-1) |
| 14 | Rebaudioside I | β-Glc-β-Glc(3-1) | β-Glc-β-Glc(2-1)-β-Glc(3-1) |
| 15 | Rebaudioside N | β-Glc-Rhaα(1-2)-β-Glc(1-3) | β-Glc-Glcβ(1-2)-β-Glc(1-3) |
| 16 | Rebaudioside O | β-Glc-[Glcβ(1-3)]Rha(1-2)Glc β(1-3) | β-Glc-β-Glc(1-2)-β-Glc-(1-3) |

As shown in Table 1, different types of steviol glycosides have a common aglycone: steviol, and the differences lie in the number and type of glycosyl groups attached at the C-13 and C-19 positions. They mainly include stevioside, rebaudioside A (Reb A), rebaudioside B, rebaudioside C, rebaudioside D, rebaudioside E, rebaudioside I, dulcoside A, steviolbioside and other glycosides.

Although steviol glycosides are high-intensity sweeteners, the lingering sweet-bitter aftertaste severely limits the application of steviol glycosides in food, beverages and other areas with special flavor requirements. The root cause of this aftertaste is the intrinsic molecular structure of steviol glycosides. The more glycosyl groups attached to the R1 and R2 groups, the better the taste. In general, the sweetness of steviosides is 110-270 times that of sucrose, and the sweetness of Rebaudioside A is 250-300 times that of sucrose. However, even in highly purified form, steviol glycosides still have undesirable taste attributes such as bitterness, sweet aftertaste, lingering sweetness, licorice flavor, etc.

The starting material containing steviol glycosides can be modified by adding more glycosyl groups at the R1 and/or R2 positions of the steviol glycoside molecule using glucosyltransferase that transfers only glucose groups in enzymatic reactions. The mechanism of action involves catalyzing the transfer of glucose residues from a glycosyl donor to a glycosyl receptor, thereby regulates the activity of the receptor molecule.

Glycosyl donors include nucleoside diphosphate (NDP) sugars such as UDP-glucose. UDP-glucose is the abbreviation of uridine diphosphate glucose, also abbreviated as UDP-Glc or UDPG, and it is a vitamin composed of uridine diphosphate and glucose. It can be considered as "active glucose" and is widely distributed in the cells of plants, animals and microorganisms. It acts as a glycosyl donor in the synthesis of sucrose, starch, glycogen and other oligosaccharides and polysaccharides, making it the most common glycosyl donor. UDP-glucosyltransferase is a type of glucosyltransferase that uses UDP-glucose as its glycosyl donor.

"Nucleoside" refers to a glycosylamine containing a nucleobase (i.e., a nitrogenous base) and a 5-carbon sugar (e.g., ribose or deoxyribose). Non-limiting examples of nucleosides include cytidine, uridine, adenosine, guanosine, thymidine, and inosine.

"Nucleoside diphosphate" refers to a glycosylamine containing a nucleobase (i.e., a nitrogenous base), a 5-carbon sugar (e.g., ribose or deoxyribose), and a diphosphate (i.e., pyrophosphate) fragment. "Nucleoside diphosphate" can be abbreviated as "NDP" Non-limiting examples of nucleoside diphosphates include cytidine diphosphate (CDP), uridine diphosphate (UDP), adenosine diphosphate (ADP), guanosine diphosphate (GDP), thymidine diphosphate (TDP), and inosine diphosphate (IDP).

In addition to UDP-glucose, other NDP-glycosyl donors include ADP-glucose, TDP-glucose, dTDP (deoxythymine diphosphate)-glucose, CDP-glucose, IDP-glucose or GDP-glucose. These glycosyl donors are expensive and are not conducive to industrial-scale production.

Glycosyl donors can be synthesized using the glycosyl donor generation system. Sucrose synthase (SUS, also abbreviated as SuSy/SS, EC2.4.1.13) catalyzes the reversible reaction of nucleotide diphosphate (NDP)-glucose and D-fructose to generate NDP and sucrose. It belongs to the glycosyltransferase-4 subfamily and was first discovered in wheat germ. Each protein exists as a tetramer. The molecular weight of each subunit is about 90kDa. The size of the gene is usually 5.9kb, and the size of the cDNA is about 2.7kb. The encoded amino acid sequence is about 800 residues in length (Ross and Davies 1992). Sucrose synthase exhibits varying affinities for different NDPs, with the order of affinity being. UDP>ADP>dTDP>CDP>GDP.

Rebaudioside A is the most abundant steviol glycoside in commercial stevia leaves. It has a high sweetness level, about 250 to 300 times that of sucrose, and it is low in calories, easy to dissolve, heat-resistant and stable. The content or purity of rebaudioside A is also a main indicator of the quality of stevia sweeteners. In addition, rebaudioside A is also an important intermediate for synthesizing other steviol glycosides, such as rebaudioside D (RebD, RD), rebaudioside E (RebE, RE), rebaudioside M (RebM, RM), rebaudioside I (RebI, RI), etc.

Rebaudioside D is another steviol glycoside with excellent taste quality and potential application prospects in the field of food and beverage. Compared with other steviol glycosides, it is sweeter, approximately 300 to 350 times that of sucrose. It has a pure sweetness and a taste resembles that of sucrose without any bitterness or licorice flavor. and it also has good stability. It is an ideal natural high-intensity sweetener.

However, the content of rebaudioside D in stevia leaves is very low (less than 5%), so the production by extraction method requires a large amount of stevia raw materials. In addition, the purification process of rebaudioside D is relatively complicated. After extraction, it needs to go through column chromatography, desalination, decolorization, recrystallization and other steps for many times. This process generates a lot of wastewater, resulting in high production costs and is not suitable for large-scale industrial production.

Rebaudioside D can be synthesized by bioenzymatic method, starting from stevia leaf extract and using glucose transferase. This biosynthetic process requires the addition of expensive UDP-glucose as one of the substrates. The process involves the action of UDP-glucosyltransferase (UGT), which catalyzes the production of rebaudioside D using stevioside or rebaudioside A as substrates. However, due to the extremely high price of UDP-glucose, the feasibility of industrial preparation of rebaudioside D is almost completely restricted, with poor economic benefits and a lack of market competitiveness.

Biosynthetic methods for converting rebaudioside molecules have been disclosed. Ohta et al. (J. Appl. Glycosci., 57, 199-209 (2010)) first reported the isolation of rebaudioside I (Reb I, RI) from stevia. Indra Prakash et al. (Molecules 2014, 19, 17345-17355) first reported the biosynthetic method for preparing RI from RA (rebaudioside A) using the glucosyltransferase UGT76G1 mutant UGT76G1-r11-f12. This method uses UDP-glucose as the glycosyl donor. In a shake flask at 30°C and pH 7.5, the reaction was carried out with MgCl2 salt, and RI yield of 22.5% was obtained. In the patent application CN106795523A with Indra Prakash as the inventor, the GenBank accession number AAR06912.1 and the DNA sequence of the glucosyltransferase UGT76G1 were disclosed. CN110914445A screened the mutants of glucosyltransferase UGT76G1 that catalyze the production of RI using RA as a raw material. The screening conditions are pH 7.0, MgCl2 10.3mM, and a temperature of 35°C. CN106795523A reports the sweetness data of RI and components containing RI. The above-mentioned prior art only discloses glucosyltransferases or their mutants that have the activity of catalyzing the synthesis of RI from RA, but these enzymes have low activity and are not suitable for industrial-scale production.

Rebaudioside M (Reb M, abbreviated as RM) has attracted more attention due to its better taste characteristics and higher price. However, its content in the dry weight of leaves is even lower than that of rebaudioside D, usually less than about 0.1%, resulting in high separation cost and high price. The method for producing high-concentration rebaudioside M by biocatalysis has attracted the attention of scholars. It has been reported that stevioside-derived recombinant enzymes can catalyze the synthesis of rebaudioside M from rebaudioside D, but the yield is relatively low. Using rebaudioside D as substrate, rebaudioside M was prepared by catalysis of microorganism-produced enzyme. Compared with traditional extraction methods, this method not only improves the production process, but also reduces environmental pollution and increases the yield of rebaudioside M.

At present, enzyme catalysis is commonly used to prepare rebaudioside M. For example, CN113186141B discloses an enzymatic method for preparing rebaudioside M, which uses sucrose, stevioside (ST) and uridine diphosphate (UDP) as raw materials. Firstly, sucrose and UDP are catalyzed by sucrose synthase from Arabidopsis thaliana to generate UDPG, and UDPG and ST are catalyzed by glycosyltransferase from Stevia rebaudiana to generate rebaudioside A; secondly, UDPG and rebaudioside A are catalyzed by glycosyltransferase from Oryza sativa to generate rebaudioside D; finally, UDPG and rebaudioside D are catalyzed by glycosyltransferase from Stevia rebaudiana to generate rebaudioside M. The yield of rebaudioside M is only about 37.9%, which is low and difficult to meet the demand for yield of rebaudioside M in industrial production. The current enzyme catalytic methods for producing rebaudioside M mainly have the following problems: (1) The cost of producing rebaudioside M from rebaudioside D using enzyme catalysis is high, and the enzyme catalytic yield needs to be further optimized; (2) The glycosyltransferases used for catalysis are difficult to separate and recover from the product, easily inactivated, and need to be supplemented, which increases the cost; (3) The content of rebaudioside A is very high in natural plants, while the content of rebaudioside D is very low. It is also a challenge to directly convert rebaudioside A into rebaudioside D at low cost.

The enzymes currently used for the bioenzymatic preparation of steviol glycosides are mainly wild-type enzymes derived from plant cells. These wild-type enzymes usually have disadvantages such as low enzyme activity and poor stability, resulting in high cost for industrial-scale production of steviol glycosides. Therefore, it is necessary to improve glucosyltransferases to obtain modified enzymes with higher enzyme activity and better stability, so as to better serve industrial-scale production.

## Summary

In order to overcome the defects of slow conversion rate and low yield in the preparation of required rebaudioside products such as rebaudioside D and rebaudioside M by enzyme catalysis in the prior art, the present invention provides a preparation method for greatly improving the required rebaudioside products such as rebaudioside D and rebaudioside M and the application of β-1,2-glucosyltransferase therein. The present invention improves the yield of the intermediate product rebaudioside D when β-1,2-glucosyltransferase catalyzes rebaudioside A to generate rebaudioside M, and then β-1,2-glucosyltransferase, β-1,3-glucosyltransferase and sucrose synthase are used in combination, which is conducive

to improving the yield of the final product rebaudioside M.

The present invention solves the above technical problems through the following technical solutions.

The first aspect of the present invention provides a β-1,2-glucosyltransferase having the amino acid sequence shown as SEQ ID NO: 4 or a mutated amino acid sequence with at least 99% sequence identity compared to the amino acid sequence shown as SEQ ID NO: 4.

In some embodiments, compared to the amino acid sequence shown as SEQ ID NO: 4, the mutated amino acid sequence comprises any four or more of the following amino acid residue differences: D323N; H324K; M181L; F185L; E188F; A196V; A198F; G201P; R373K; L375M; L325F; V326A. For another example, compared to the amino acid sequence shown as SEQ ID NO: 4, the mutated amino acid sequence comprises: four or five of D323N; H324K; M181L; F185L; E188F; A196V; A198F; G201P; R373K; L375M; L325F; V326A.

In some embodiments, compared to the amino acid sequence shown as SEQ ID NO: 4, the mutated amino acid sequence comprises: D323N, H324K and any two or more of the following amino acid residue differences: M181L; F185L; E188F; A196V; A198F; G201P; R373K; L375M; L325F; V326A. For another example, compared to the amino acid sequence shown as SEQ ID NO: 4, the mutated amino acid sequence comprises: D323N, H324K, and two or three of the following amino acid residue differences: M181L; F185L; E188F; A196V; A198F; G201P; R373K; L375M; L325F; V326A.

In some embodiments, the mutated amino acid sequence comprises the following amino acid residue differences compared to the amino acid sequence shown as SEQ ID NO:4: D323N, H324K, M181L, F185L and E188F.

In other embodiments, the mutated amino acid sequence comprises the following amino acid residue differences compared to the amino acid sequence shown as SEQ ID NO:4: D323N, H324K, A196V, A198F and G201P.

In other embodiments, the mutated amino acid sequence comprises the following amino acid residue differences compared to the amino acid sequence shown as SEQ ID NO:4: D323N, H324K, R373K and L375M.

In other embodiments, the mutated amino acid sequence comprises the following amino acid residue differences compared to the amino acid sequence shown as SEQ ID NO:4: D323N, H324K, L325F and V326A.

In some specific embodiments, the mutant amino acid sequence is shown as SEQ ID NO:16, SEQ ID NO:18, SEQ ID NO:20 or SEQ ID NO:22.

The second aspect of the present invention provides an isolated nucleic acid molecule encoding the β-1,2-glucosyltransferase as described in the first aspect.

In some embodiments, the nucleotide sequence of the nucleic acid molecule is shown as SEQ ID NO:3, SEQ ID NO:15, SEQ ID NO:17, SEQ ID NO:19 or SEQ ID NO:21.

The third aspect of the present invention provides a recombinant expression vector, which comprises a backbone vector, and the isolated nucleic acid molecule as described in the second aspect integrated into the backbone vector.

In some specific embodiments, the backbone vector comprises a phage vector, a plasmid vector or a viral vector. The plasmid vector comprises pET28a or pET21a.

The fourth aspect of the present invention provides a transformant, which is a genetically recombinant cell obtained by the introduction of the isolated nucleic acid molecule as described in the second aspect or the recombinant expression vector as described in the third aspect into a host cell.

In some embodiments, the host cell is a eukaryotic cell or a prokaryotic cell.

In some embodiments, the prokaryotic cell is *Escherichia coli,* for example, BL21(DE3).

In some embodiments, the transformant is selected from any one or more of the following strains numbered as RMIS708, RMIS828, RMIS829, RMIS830 and RMIS831 of Abiochem Biotechnology (Shanghai) Co., Ltd.

The fifth aspect of the present invention provides a method for preparing the β-1,2-glucosyltransferase as described in the first aspect, comprising culturing the transformant as described in the fourth aspect in a culture medium to obtain the β-1,2-glucosyltransferase.

In some embodiments, the culture medium is selected from LB liquid medium and/or TB liquid medium.

In some embodiments, the condition for the culturing is: shaking culture at 37±1°C.

The sixth aspect of the present invention provides a preparation method of rebaudioside D, comprising the step of combining at least rebaudioside A, a glycosyl donor and the β-1,2-glucosyltransferase as described in the first aspect to form a reaction system and proceeding the reaction.

In the present invention, the reaction system refers to the solution system in which all reaction components involved in the reaction are located during the reaction process. In the embodiment of the sixth aspect of the present invention, the reaction system includes components such as a crude enzyme solution or a reaction enzyme solution containing β-1,2-glucosyltransferase, a substrate, a product and a solvent. The same applies to other embodiments.

In some embodiments, the β-1,2-glucosyltransferase is used in the form of wet cells, bacterial powder, liquid enzyme, solid enzyme powder or immobilized enzyme.

In the present invention, wet cells refer to the precipitate obtained by centrifuging the culture broth of bacteria expressing glycosyltransferase and discarding the supernatant. Bacterial powder refers to the powder obtained by drying the above wet cells to remove the solvent water and grinding it. Liquid enzyme (such as crude enzyme solution) refers to the enzyme solution obtained by resuspending the above wet cells with a solvent (such as water) at a certain proportion,

preferably the supernatant obtained by homogenizing the resuspended enzyme solution at a certain pressure and centrifuging it at a certain speed. Reaction enzyme solution refers to the liquid enzyme that is stood at a constant temperature of 80±10°C for a period of time to remove impurity enzymes. Solid enzyme powder refers to the powder obtained by drying and grinding the above liquid enzyme. Immobilized enzyme refers to the enzyme components from the above liquid enzyme immobilized onto an immobilized carrier. Immobilized carriers include resins, such as epoxy resins, amino resins and adsorption resins. More specifically, the epoxy resin can be SEPABEADS®ECHFA, ReliZymeTMHFA403, ReliZymeTMEP113, ReliZymeTMEP403 and/or SEPABEADS®ECEP.

In some embodiments, the addition amount of rebaudioside A in the reaction system is 20-120 g/L, preferably 40-110 g/L, and more preferably 50-100 g/L.

In some embodiments, the glycosyl donor is NDP-glucose; NDP is preferably selected from any one or more of adenosine diphosphate (ADP), uridine diphosphate (UDP), cytidine diphosphate (CDP), guanosine diphosphate (GDP), thymidine diphosphate (TDP) and inosine diphosphate (IDP).

In some embodiments, the pH of the reaction is 5.0-8.0, preferably 6.0.

In some embodiments, the temperature of the reaction is 20-90°C, preferably 60°C.

In some embodiments, the time of the reaction is 10 minutes to 24 hours, preferably 30 minutes.

In some embodiments, the β-1,2-glucosyltransferase is used in the form of wet cells, and the ratio of the added mass of the wet cells to rebaudioside A in the reaction system can be (0.1-0.8):1, or (0.12-0.6):1, or (0.24-0.3):1.

In the present invention, the added mass refers to the initial mass of components such as wet cells or substrates when added into the reaction system.

In the present invention, an example of preparing the crude enzyme solution is resuspending the wet cells expressing the β-1,2-glucosyltransferase using a buffer solution at a ratio of 1:5 (g/mL), then homogenizing the resuspension at 550 bar for 1.5 min (to lyse the bacteria), and after centrifugation at 12000 rpm for 2 min, collecting the supernatant to obtain the crude enzyme solution.

In the present invention, the buffer solution can be a phosphate buffer with a pH value of 6.0.

In some embodiments, the added amount of wet cells in the reaction system can be 6-30 g/L, or 12-24 g/L.

In some embodiments, the β-1,2-glucosyltransferase is used in the form of liquid enzyme; the liquid enzyme is preferably a crude enzyme solution or a reaction enzyme solution; the crude enzyme solution is obtained from wet cells expressing the β-1,2-glucosyltransferase; the reaction enzyme solution is obtained by standing the crude enzyme solution at a constant temperature of 80±10°C for a period of time.

In some embodiments, the crude enzyme solution is a homogenous enzyme solution obtained by resuspension and homogenization of the wet cells with a solvent, and the ratio of the added mass of the wet cells to the added volume of the solvent is 1g:(5-10)mL. For example, the crude enzyme solution is a homogenous enzyme solution obtained by resuspension and homogenization of the wet cells with a solvent, and the ratio of the added mass of the wet cells to the added volume of the solvent is 1g:5mL. The solvent is water or a buffer solution (for example, a phosphate buffer). For another example, the ratio of the added mass of the wet cells to the added volume of the solvent can also be 1g:10mL.

In some embodiments, the ratio of the added volume of the crude enzyme solution to the added mass of rebaudioside A in the reaction system can be (0.5-5):1, (0.6-3):1, or (1.2-1.5):1 mL/g.

The seventh aspect of the present invention provides an enzyme combination system, which comprises: the β-1,2-glucosyltransferase as described in the first aspect, wherein the β-1,2-glucosyltransferase is capable of catalyzing the reaction of rebaudioside A with a glycosyl donor to produce rebaudioside D; and a β-1,3-glucosyltransferase, wherein the β-1,3-glucosyltransferase is capable of catalyzing the reaction of rebaudioside D with a glycosyl donor to produce rebaudioside M.

In some embodiments, the β-1,2-glucosyltransferase is used in the form of wet cells, for example, referred to as the second wet cells; the β-1,3-glucosyltransferase is used in the form of wet cells, for example referred to as the third wet cells. The relevant definitions of the wet cells are as described above.

In some embodiments, the enzyme combination system also comprises: a glycosyl donor synthase, which is capable of catalyzing the reaction of a sugar with NDP to produce the glycosyl donor. In some embodiments, the glycosyl donor synthase is used in the form of wet cells, for example, referred to as the first wet cells.

In some embodiments, the sugar is sucrose, the glycosyl donor is NDP-glucose, and the glycosyl donor synthase is sucrose synthase.

In some embodiments, the glycosyl donor synthase is obtained by fermentation cultivation of the first transformant. The first transformant is a genetically recombinant cell obtained by introducing the nucleotide sequence shown as SEQ ID NO: 1 into the host cell, or a genetically recombinant cell capable of expressing the amino acid sequence shown as SEQ ID NO: 2.

In some embodiments, the β-1,2-glucosyltransferase is obtained by fermentation cultivation of the second transformant; the second transformant is a genetically recombinant cell obtained by introducing the isolated nucleic acid molecule described in the second aspect or the recombinant expression vector described in the third aspect into the host cell.

In some embodiments, the β-1,3-glucosyltransferase is obtained by fermentation cultivation of the third transformant; the third transformant is a genetically recombinant cell obtained by introducing the nucleotide sequence shown as SEQ ID NO:23 into the host cell, or a genetically recombinant cell capable of expressing the amino acid sequence shown as SEQ ID NO:24.

In some embodiments, the first transformant is the strain numbered as RMIS609 of Abiochem Biotechnology (Shanghai) Co., Ltd.

In some embodiments, the second transformant is selected from any one or more of the strains numbered as RMIS708, RMIS828, RMIS829, RMIS830 and RMIS831 of Abiochem Biotechnology (Shanghai) Co., Ltd.

In some embodiments, the third transformant is a strain numbered as RMIS109 of Abiochem Biotechnology (Shanghai) Co., Ltd.

The eighth aspect of the present invention provides a preparation method of rebaudioside M, which comprises: the step of combining rebaudioside A, a glycosyl donor and the enzyme combination system as described in the seventh aspect to form an enzyme catalytic system and proceeding the reaction (i.e., a one-pot reaction); or the step of combining rebaudioside A, a glycosyl donor and β-1,2-glucosyltransferase to form an enzyme catalytic system and proceeding the reaction, then adding β-1,3-glucosyltransferase and continuing the reaction (i.e., a step-by-step reaction).

In some embodiments, the β-1,2-glucosyltransferase is the β-1,2-glucosyltransferase described in the first aspect of the present invention.

In some embodiments, the β-1,2-glucosyltransferase and/or the β-1,3-glucosyltransferase are used in the form of wet cells, bacterial powder, liquid enzyme, solid enzyme powder or immobilized enzyme.

In some embodiments, the added amount of rebaudioside A in the enzyme catalytic system can be 20-120 g/L, 40-110 g/L, or 50-100 g/L.

In some embodiments, the glycosyl donor in the reaction system is NDP-glucose; NDP is selected from any one or more of adenosine diphosphate (ADP), uridine diphosphate (UDP), cytidine diphosphate (CDP), guanosine diphosphate (GDP), thymidine diphosphate (TDP) and inosine diphosphate (IDP).

In some embodiments, the pH of the reaction is 5.0-8.0, preferably 6.0.

In some embodiments, the temperature of the reaction is 20-90°C, preferably 60°C.

In some embodiments, the time of the reaction is 10 minutes to 24 hours, preferably 30 minutes to 6 hours.

In some embodiments, the β-1,2-glucosyltransferase is used in the form of wet cells. The ratio of the added mass of the wet cells to rebaudioside A in the enzyme catalytic system can be (0.1-0.8):1, (0.12-0.6):1, or (0.24-0.3):1.

In some embodiments, the added amount of the wet cells in the enzyme catalytic system can be 6-30 g/L, or 12-24 g/L.

In some embodiments, the β-1,2-glucosyltransferase is used in the form of a liquid enzyme; the liquid enzyme is preferably a crude enzyme solution or a reaction enzyme solution; the crude enzyme solution is prepared from the second wet cells expressing the β-1,2-glucosyltransferase; the reaction enzyme solution is obtained by standing the crude enzyme solution at a constant temperature of 80±10°C for a period of time.

In some embodiments, the crude enzyme solution is a homogenized enzyme solution obtained by resuspension and homogenization of the wet cells with a solvent, and the ratio of the added mass of the wet cells to the added volume of the solvent is 1 g: (5-10) mL.

In some embodiments, the ratio of the added volume of the crude enzyme solution to the added mass of rebaudioside A in the enzyme catalytic system can be (0.5-5):1, or (0.6-3):1, or (1.2-1.5):1 mL/g.

In some embodiments, the wet cells expressing the β-1,2-glucosyltransferase or the liquid enzyme containing the β-1,2-glucosyltransferase is prepared from the second transformant capable of expressing the β-1,2-glucosyltransferase; preperably, the second transformant is selected from any one or more of the strains numbered as RMIS708, RMIS828, RMIS829, RMIS830, and RMIS831 of Abiochem Biotechnology (Shanghai) Co., Ltd.

In some embodiments, the β-1,3-glucosyltransferase is used in the form of wet cells, and the ratio of the added mass of the wet cells to the rebaudioside A in the enzyme catalytic system can be (0.1-0.8):1, or (0.12-0.6):1, or (0.24-0.3):1.

In some embodiments, the added amount of the wet cells in the enzyme catalytic system can be 6-30 g/L, or 12-30 g/L.

In some embodiments, the β-1,3-glucosyltransferase is used in the form of a liquid enzyme; the liquid enzyme is preferably a crude enzyme solution or a reaction enzyme solution; the crude enzyme solution is prepared from the third wet cells expressing the β-1,3-glucosyltransferase; the reaction enzyme solution is obtained by standing the crude enzyme solution at a constant temperature of 80±10°C for a period of time.

In some embodiments, the crude enzyme solution is a homogenized enzyme solution obtained by resuspension and homogenization of the wet cells with a solvent, and the ratio of the added mass of the wet cells to the added volume of the solvent is 1 g: (5-10) mL.

In some embodiments, the ratio of the added volume of the crude enzyme solution to the added mass of rebaudioside A in the enzyme catalytic system can be (0.5-5):1, or (0.6-3):1, or (1.2-1.5):1 mL/g.

In some embodiments, the wet cells expressing the β-1,3-glucosyltransferase or the liquid enzyme containing the β-1,3-glucosyltransferase is prepared from the third transformant capable of expressing the β-1,3-glucosyltransferase; preferably, the third transformant is the strain numbered as RMIS109 of Abiochem Biotechnology (Shanghai) Co., Ltd.

In some embodiments, the glycosyl donor synthase is used in the form of wet cells, and the ratio of the added mass of the wet cells to the rebaudioside A in the enzyme catalytic system can be (0.1-0.8):1, or (0.12-0.6):1, or (0.24-0.3):1.

In some embodiments, the added amount of the wet cells in the enzyme catalytic system can be 6-30g/L, also can be 12-30g/L.

In some embodiments, the glycosyl donor synthase is used in the form of a liquid enzyme; the liquid enzyme is preferably a crude enzyme solution or a reaction enzyme solution; the crude enzyme solution is prepared from the first transformant capable of expressing the glycosyl donor synthase; the reaction enzyme solution is obtained by standing the crude enzyme solution at a constant temperature of 80±10° C for a period of time. In some embodiments, the glycosyl donor synthase is sucrose synthase.

In some embodiments, the crude enzyme solution is a homogenized enzyme solution obtained by resuspension and homogenization of the wet cells with a solvent, and the ratio of the added mass of the wet cells to the added volume of the solvent is 1 g: (5-10) mL.

In some embodiments, the ratio of the added volume of the crude enzyme solution to the added mass of rebaudioside A in the enzyme catalytic system can be (0.5-5):1, or (0.6-3):1, or (1.2-1.5):1 mL/g.

In some embodiments, the wet cells expressing the glycosyl donor synthase in the enzyme combination system or the liquid enzyme containing the glycosyl donor synthase is prepared from the first transformant capable of expressing the glycosyl donor synthase; preferably, the first transformant is the strain numbered as RMIS609 of Abiochem Biotechnology (Shanghai) Co., Ltd.

The ninth aspect of the present invention provides the use of the β-1,2-glucosyltransferase as described in the first aspect or the enzyme combination system as described in the seventh aspect in preparing rebaudioside D or rebaudioside M with rebaudioside A and a glycosyl donor as substrates.

The tenth aspect of the present invention provides an enzyme-catalyzed reaction solution, which comprises: rebaudioside A, glycosyl donor, rebaudioside D, and the β-1,2-glucosyltransferase described in the first aspect.

In some embodiments, the enzyme-catalyzed reaction solution also comprises β-1,3-glucosyltransferase and rebaudioside M.

In some embodiments, the enzyme-catalyzed reaction solution also comprises: sucrose synthase, NDP, sucrose, NDP-glucose and D-fructose.

The eleventh aspect of the present invention provides an enzyme-catalyzed final product system comprising: rebaudioside A; rebaudioside D; rebaudioside M; and the β-1,2-glucosyltransferase capable of catalyzing the conversion of rebaudioside A and a glycosyl donor to produce the rebaudioside D. Wherein the β-1,2-glucosyltransferase is the β-1,2-glucosyltransferase described in the present invention; and a β-1,3-glucosyltransferase capable of catalyzing the conversion of rebaudioside D and a glycosyl donor to produce the rebaudioside M.

In some embodiments, in the enzyme-catalyzed final product system, the content of rebaudioside A accounts for less than 1% of the total content of rebaudioside A, rebaudioside D, and rebaudioside M; the content of rebaudioside D accounts for less than 9.5% of the total content of rebaudioside A, rebaudioside D and rebaudioside M, and the content of rebaudioside M accounts for more than 84% of the total content of rebaudioside A, rebaudioside D and rebaudioside M.

In some embodiments, in the enzyme-catalyzed final product system, the content of rebaudioside A accounts for less than or equal to 0.88% of the total content of rebaudioside A, rebaudioside D and rebaudioside M. Further, in the enzyme-catalyzed final product system, the content of rebaudioside A accounts for less than or equal to 0.36% of the total content of rebaudioside A, rebaudioside D and rebaudioside M.

In some embodiments, in the enzyme-catalyzed final product system, the content of rebaudioside D accounts for less than or equal to 7.42% of the total content of rebaudioside A, rebaudioside D and rebaudioside M. Further, in the enzyme-catalyzed final product system, the content of rebaudioside D accounts for less than or equal to 3.57% of the total content of rebaudioside A, rebaudioside D and rebaudioside M.

In some embodiments, in the enzyme-catalyzed final product system, the content of rebaudioside M accounts for greater than or equal to 92.22% of the total content of rebaudioside A, rebaudioside D and rebaudioside M. Further, in the enzyme-catalyzed final product system, the content of rebaudioside M accounts for greater than or equal to 95.55% of the total content of rebaudioside A, rebaudioside D and rebaudioside M.

The twelfth aspect of the present invention provides a composition comprising rebaudioside D, which is prepared by the preparation method described in the sixth aspect of the present invention.

The thirteenth aspect of the present invention provides a composition comprising rebaudioside M, which is prepared by the preparation method described in the eighth aspect of the present invention.

The fourteenth aspect of the present invention provides a composition comprising rebaudioside D and rebaudioside M, which is prepared by the preparation method described in the eighth aspect of the present invention.

The fifteenth aspect of the present invention provides a composition comprising: rebaudioside A; rebaudioside D; rebaudioside M; wherein, the content of rebaudioside A accounts for less than 1% of the total content of rebaudioside A, rebaudioside D and rebaudioside M, the content of rebaudioside D accounts for less than 9.5% of the total content of rebaudioside A, rebaudioside D and rebaudioside M, and the content of rebaudioside M accounts for more than 84% of the total content of rebaudioside A, rebaudioside D and rebaudioside M.

In some embodiments, in the composition, the content of rebaudioside A accounts for less than or equal to 0.88% of the total content of rebaudioside A, rebaudioside D and rebaudioside M. Further, in the composition, the content of rebaudioside A accounts for less than or equal to 0.36% of the total content of rebaudioside A, rebaudioside D and rebaudioside M.

In some embodiments, in the composition, the content of rebaudioside D accounts for less than or equal to 7.42% of the total content of rebaudioside A, rebaudioside D and rebaudioside M. Further, in the composition, the content of rebaudioside D accounts for less than or equal to 3.57% of the total content of rebaudioside A, rebaudioside D and rebaudioside M.

In some embodiments, in the composition, the content of rebaudioside M accounts for greater than or equal to 92.22% of the total content of rebaudioside A, rebaudioside D, and rebaudioside M. Further, in the composition, the content of rebaudioside M accounts for greater than or equal to 95.55% of the total content of rebaudioside A, rebaudioside D, and rebaudioside M.

The sixteenth aspect of the present invention provides a method for producing the target steviol glycoside (including rebaudioside D or rebaudioside M, or a composition of rebaudioside D and rebaudioside M), comprising the following steps:

(a) providing a source of steviol glycoside;
(b) providing a glycosyltransferase;
(c) providing a glycosyl donor or a glycosyl donor generation system;
(d) preparing a reaction mixture using (i) steviol glycoside, (ii) glycosyltransferase and (iii) glycosyl donor or glycosyl donor generation system, and proceeding the reaction at a temperature of about 30° C to 90° C; wherein steps (a)-(c) above have no specific order;
(e) making the distribution change of the target steviol glycoside reach 50% or more within 2 hours to form a precipitate containing the target steviol glycoside, more preferably, reach 60%, 70%, 80% or 90% or more within 2 hours; optionally, the method also includes:
(f) separating and purifying a precipitate containing the target steviol glycoside from the reaction mixture, the target steviol glycoside is preferably rebaudioside D and/or rebaudioside M.

The target steviol glycoside means that when the prepared product is mainly Reb D, the target steviol glycoside is Reb D; when the prepared product is mainly Reb M, the target steviol glycoside is Reb M; when the prepared product is mainly a mixture of Reb D and Reb M, the target steviol glycoside is Reb D and Reb M.

In some embodiments, the source of steviol glycoside is selected from one or more of the following groups: stevia leaf extract, rubusoside, stevioside, rebaudioside A and rebaudioside D.

In some embodiments, the steviol glycoside contains at least 60 wt%, 70 wt%, 80 wt%, 90 wt%, 95 wt%, 97 wt%, 98 wt% or more of rebaudioside A.

In some embodiments, the glycosyltransferase is selected from one or more of the following groups: β-1,2-glycosyltransferases and β-1,3-glycosyltransferases.

In some embodiments, the β-1,2 glycosyltransferase is a glycosyltransferase that performs β-1,2 glycosylation on the C2' position of 13-O-glucose, 19-O-glucose, or 13-O-glucose and 19-O-glucose in steviol glycosides; and the β-1,3-glycosyltransferase is a glycosyltransferase that performs β-1,3-glycosylation on the C3' position of 13-O-glucose, 19-O-glucose, or 13-O-glucose and 19-O-glucose in steviol glycosides.

In some embodiments, the glycosyltransferase is thermostable.

Thermostable means that the glycosyltransferase can react at 55°C to 65°C for 4-24 hours.

In some embodiments, the glycosyl donor comprises an NDP-sugar; the NDP-sugar is selected from ADP-glucose, GDP-glucose, CDP-glucose, UDP-glucose, TDP-glucose, IDP-glucose or a combination thereof.

In some embodiments, the glycosyl donor generation system comprises sucrose, a glycosyl donor synthase and NDP.

In some embodiments, the glycosyl donor synthase is thermostable.

In some embodiments, at the beginning of the reaction, the molar ratio of sucrose to the steviol glycoside in the reaction mixture is 4:1 to 10:1, preferably 4.5:1 to 5:1;

and/or the concentration of sucrose is 100 g/L to 200 g/L, preferably 130 g/L to 140 g/L;

and/or the concentration of the steviol glycoside is 20 g/L to 120 g/L, preferably 70 g/L to 80 g/L;
and/or the reaction temperature is 55°C to 65°C, preferably 60°C;
and/or the reaction pH is 5.0 to 8.0, preferably 6.0 to 6.2.

In some embodiments, the pH is reched by adding a food-grade acid.
In some embodiments, the food-grade acid is selected from the following groups: phosphoric acid, acetic acid, and citric acid.
In some embodiments, the amino acid sequence of the β-1,3-glycosyltransferase has at least about 90% identity with SEQ ID NO: 24; and/or the β-1,2-glycosyltransferase is the β-1,2-glucosyltransferase described in the first aspect of the present invention.
In some embodiments, the nucleotide sequence of the gene encoding the β-1,3-glycosyltransferase has at least about 90% identity with SEQ ID NO: 23; and/or the gene encoding the β-1,2 glycosyltransferase is the nucleic acid molecule described in the second aspect of the present invention.
In some embodiments, the amino acid sequence of the glycosyl donor synthase has at least about 90% identity with SEQ ID NO:2.
In some embodiments, the nucleotide sequence of the gene encoding the glycosyl donor synthase has at least 90% identity with SEQ ID NO: 1.
In some embodiments, the distribution change of the steviol glycoside reaches 85% or more within 30 minutes to 4 hours after preparing the reaction mixture.
The seventeenth aspect of the present invention provides a consumable comprising rebaudioside D and/or rebaudioside M, which is prepared by the method described in the sixteenth aspect of the present invention.
On the basis of common knowledge in the field, the above preferred conditions may be arbitrarily combined to obtain preferred examples of the present invention.
The reagents and raw materials used in the present invention are all commercially available.
The positive and progressive effect of the present invention is that the present invention employs an enzyme catalytic method to catalyze the reaction of rebaudioside A and a glycosyl donor to generate rebaudioside D, and then generate rebaudioside M. Since the present invention optimizes the β-1,2-glucosyltransferase, the yield of the intermediate product stevioside D is improved during the catalytic conversion of rebaudioside A into rebaudioside M, which is conducive to increasing the yield of the final product rebaudioside M.

## Brief description of the drawings

FIG. 1 is the reaction route for the synthesis of Reb M; wherein, β-1,2-GT: β-1,2-glycosyltransferase; β-1,3-GT: β-1,3-glycosyltransferase; SUS: sucrose synthase.
FIG. 2 is the graph showing the distribution of RebA, RebD and RebM over time during the reaction.

## Detailed description of the invention

The present invention is further illustrated below by way of examples, but it is not limited to the scope of these examples. Experimental methods that do not indicate specific conditions in the following examples shall be selected according to conventional methods and conditions, or according to the product's instruction manual.
The following three enzymes are used for the enzymatic conversion process in the following examples.

1. β-1,2-glycosyltransferase promotes glucose transfer reactions in the presence of uridine diphosphate glucose (UDPG) or adenosine diphosphate glucose (ADPG). Reb A (SvG1G3) is converted into Reb D (SvG2G3), in which an additional glucose unit is linked via a β-1,2 covalent bond to the C-19 position of the stevia aglycone;
2. In the presence of the glycosyl donor UDPG or ADPG, β-1,3-glycosyltransferase promotes the transfer of glucose to Reb D (SvG2G3) to generate Reb M (SvG3G3);
3. In the presence of sucrose, the glucose group is transferred to uridine diphosphate ("UDP") or adenosine diphosphate ("ADP") via sucrose synthase to produce UDPG or ADPG. The reaction is reversible, and the UDP-UDPG circular reaction can be repeated.

Efficient production of Reb M can be achieved when the β-1,3-glycosyltransferase and β-1,2-glycosyltransferase-mediated glycosyltransfer reactions are coupled with sucrose synthasemediated UDP-UDPG or ADP-ADPG circular reaction. The reaction route is shown in FIG. 1.

Distribution change of steviol glycoside:

The "distribution change of steviol glycoside" as used herein refers to the increase or decrease in the relative proportion of the target steviol glycoside in the final product compared to its relative proportion in the starting material. For example, if the proportion of Reb M in the starting material is 0 and the proportion of Reb M in the final product is 95%, then the change is 95%.

When producing high-Reb M products, a useful distribution change of steviol glycoside reaches 50% or more in 2 hours, preferably reaches 60%, 70%, 80%, and 90% or more within 2 hours

The target distribution may vary, in some examples, Reb M accounts for more than 50% by peak area in the conversion product, more preferably 95% or more by peak area. In some cases, a Reb M concentration of 95% or more by peak area can be achieved in less than 8 hours, preferably less than 5 hours, and less than 3 hours in some examples.

The present invention aims to achieve the target distribution in a shorter time compared to conventional reactions. In some cases, the target distribution of steviol glycoside may be achieved within 8 h or less, more ideally within about 4 h after the start of the reaction.

The reagents used in the present invention are shown as follows:
Rebaudioside A (RA) was purchased from Shanghai yuanye Bio-Technology Co., Ltd.

RA60 was purchased from Zhucheng Haotian Pharm Co., Ltd.

Sucrose was purchased from Sangon Biotech (Shanghai) Co., Ltd.

*E.coli* Trans10 competent cells, *E. coli* BL21 (DE3) competent cells were purchased from Beijing Dingguo Changsheng Biotechnology Co., Ltd.

Composition of LB liquid medium: 10 g/L of tryptone, 5 g/L of yeast extract, 10 g/L of NaCl.

Composition of TB liquid medium: 2% trypsin, 2.4% yeast extract, 72 mM $K_2HPO_4$, 17 mM $KH_2PO_4$, 0.4% glycerol.

pET28a plasmid and recombinant plasmids were synthesized by Sangon Biotech (Shanghai) Co., Ltd.

The HPLC assay method is as follows:
Chromatographic column: Diamonsil C18 (2) (4.6 mm 250 mm, 5 μm); mobile phase A: weigh 1.5 g sodium dihydrogen phosphate ($NaH_2PO_4$), then dissolve it in 1000 mL of water and adjust pH to 2.6 with phosphoric acid; mobile phase B: acetonitrile; gradient elution was performed according to the conditions listed in Table 2. Detection wavelength: 210 nm; flow rate: 0.8 mL/min; injection volume: 20 μL; column temperature: 40°C. The retention time of RA is 21.905min; the retention time of RD is 12.614min; the retention time of RM is 16.458min.

Table 2 HPLC gradient elution conditions

| Time (min) | Mobile phase A% | Mobile phase B% |
|---|---|---|
| 0.00 | 75 | 25 |
| 13.0 | 75 | 25 |
| 15.0 | 68 | 32 |
| 27.0 | 68 | 32 |
| 40.0 | 30 | 70 |
| 45.0 | 30 | 70 |
| 45.5 | 75 | 25 |
| 60.0 | 75 | 25 |

Among them, when the final product RM is generated using RA as the substrate, the actual concentration of RM ($C_{RM}$) is calculated according to the external standard method and the standard substance.

The calculation method of the yield of RM (RM%) is as follows:

Yield of RM = [(actual concentration of generated RM ($C_{RM}$))/theoretical concentration of generated RM] × 100%

Theoretical concentration of generated RM = [(concentration of substrate RA ($C_{RM}$))/(molecular weight of substrate RA ($M_{RA}$))] × molecular weight of RM ($M_{RM}$).

If using RA as the substrate to generate RD, the actual concentration of generated RD ($C_{RD}$) And the yield of RD (RD%) can be calculated in the same way.

### Example 1: Preparation of the reaction enzyme solution of GT609

The present example relates to the preparation method of the GT609 reaction enzyme solution. GT609 belongs to sucrose synthase (Sucrose synthase, SUS, also abbreviated as SuSy / SS, EC 2.4.1.13). It can catalyze the transfer of hexose group. The reaction equation is NDP + sucrose → NDP-glucose + D-fructose, so it is also one of glycosyltransferases. NDP is an abbreviated form of nucleoside diphosphate, including adenosine diphosphate (ADP), uridine diphosphate (UDP), cytidine diphosphate (CDP), guanosine diphosphate (GDP), thymidine diphosphate (TDP) and inosine diphosphate (IDP), etc. Accordingly, NDP-glucose includes ADP-glucose, UDP-glucose, CDP-glucose, GDP-glucose, TDP-glucose, and IDP-glucose, etc. NDP-glucose is able to serve as a glycosyl donor in subsequent reactions.

The GT609 of this example is Enzyme 14 (Enz.14) as described in paragraph 0128 of the specification of the invention patent application with the publication number CN115678867A, the nucleotide sequence of GT609 is shown as SEQ ID NO: 1, and the amino acid sequence of GT609 is shown as SEQ ID NO: 2.

The method of obtaining the GT609 reaction enzyme solution provided in this example:

1.1. The nucleotide sequence of GT609 (as shown in SEQ ID NO: 1) was synthesized by Sangon Biotech (Shanghai) Co., Ltd. and integrated into the pET28a plasmid vector to obtain the recombinant plasmid pET28a GT609.

1.2. The recombinant plasmid pET28a GT609 was transformed into the host *E. coli* BL21 (DE3) competent cells to obtain a genetically engineered strain (designated RMIS609) containing the recombinant plasmid pET28a-GT609). Single colonies of the genetically engineered strain were picked and inoculated into 5 mL of LB liquid medium containing 50 μg/mL kanamycin and shaking cultured at 37°C for 4 h. Then the culture was transferred to 50 mL of freshly prepared TB liquid medium containing 50 μg/mL kanamycin at an inoculum amount of 2v/v% (volume percentage) and shaking cultured at 37°C until $OD_{600}$ reached about 0.8. IPTG was added to a final concentration of 0.1 mM, and the culture was induced at 25°C for 20 h. After the culture is completed, the culture broth was centrifuged at 4000 rpm for 20 min, then the supernatant was discarded and the precipitate was collected to obtain RMIS609 wet cells. The wet cells were stored at -20°C for future use.

1.3. The collected RMIS609 wet cells were suspended in PBS (50 mM, pH 6.0) at a ratio of 1 g: 5 mL (mass / volume ratio, namely M / V, also referred to as homogenization ratio) to obtain the suspension. Then, the suspension was homogenized using a high-pressure homogenizer to obtain the homogeneous enzyme solution. The condition for homogenization was homogenization at a pressure of 550 MPa for 1.5 min. The homogenized enzyme solution was centrifuged at 12,000 rpm for 2 min, then the precipitation was discarded, and the supernatant was collected to obtain GT609 crude enzyme solution.

1.4. GT609 crude enzyme solution was stand at a constant temperature of 80°C to obtain GT609 reaction enzyme solution.

### Example 2: Preliminary screening of β -1,2-glucosyltransferase

β-1,2-glucosyltransferase (abbreviated as β-1,2-GT) can catalyze the reaction of rebaudioside A and a glycosyl donor to generate rebaudioside D (RD).

After screening enzymes in our own enzyme library (the enzyme screening reaction system is shown in Table 3, wherein the preparation methods of the crude enzyme solution and the reaction enzyme solution of β-1,2-glucosyltransferase are the same as those described in Example 1), β-1,2-glucosyltransferase GT708 with high catalytic activity in the reaction of converting rebaudioside A into rebaudioside D was finally selected. The nucleotide sequence of GT708 is shown as SEQ ID NO: 3, and its amino acid sequence is shown as SEQ ID NO: 4.

The preparation methods of both the GT708 crude enzyme solution and the GT708 reaction enzyme solutions provided in this example were the same as those described in Example 1. The difference was that the recombinant plasmid obtained was pET28a-GT708. The resulting genetically engineered strain containing the recombinant plasmid pET28a-GT708 was designated as RMIS708.

Table 3 Composition of the enzyme screening reaction system

| Component | Concentration of mother liquor | Addition amount | Final concentration |
|---|---|---|---|
| GT609 reaction enzyme solution | Stock solution | 30 μL | / |
| β-1,2-glucosyltransferase reaction enzyme solution | Stock solution | 150 μL | / |
| Sucrose | 400 g/L | 500 μL in total | 200 g/L |
| Rebaudioside A | 100 g/L | | 50 g/L |

(continued)

| Component | Concentration of mother liquor | Addition amount | Final concentration |
|---|---|---|---|
| NDP (ADP or UDP) | 10 g/L | 10 μL | 0.1 g/L |
| PBS buffer | 50 mM, pH 6.0 | allowance | / |
| Total volume of the reaction system | 1 mL | | |

In Table 3, the mother liquor concentration refers to the initial concentration of each component at the beginning of addition. Stock solution refers to the GT609 reaction enzyme solution or the β-1-2-glucosyltransferase reaction enzyme solution was added in an undiluted state. The final concentration refers to the initial concentration of each component in the whole reaction system after addition, which is the concentration at the reaction time of 0h. NDP (ADP or UDP) refers to the reaction system using ADP and the reaction system using UDP were respectively prepared by using different NDP. Therefore, Table 3 includes two reaction systems.

The reaction mechanism of substances listed in Table 3 is as follows. Sucrose and NDP are converted into NDP-glucose and D-fructose by sucrose synthase (SUS) in the GT609 reaction enzyme solution. NDP-glucose (as a glycosyl donor) and rebaudioside A (RA) are converted into rebaudioside D (RD) under the action of β-1,2-glucosyltransferase (β-1,2-GT) in the β-1,2-glucosyltransferase reaction enzyme solution.

The enzyme screening method in this example comprises the following steps:

The reaction system was prepared according to Table 3. Then it was placed in a metal bath (at a set heating temperature of 60°C) and reacted at 60°C for 30 min to obtain the reaction solution. The reaction solution was diluted by 50-fold and sampled for HPLC analysis.

Based on the results of HPLC and the dilution factor of the reaction solutions, GT708 was found to be the most effective among the screened β-1,2-glucosyltransferases. For the reaction system using ADP, its RD yield was 66.606%. For the reaction system using UDP, its RD yield was 28.842%.

**Example 3: Mutation of β-1,2-glucosyltransferase GT708**

According to the results of Example 2, when sucrose and NDP were used as starting materials, and GT708 reaction enzyme solution and GT609 reaction enzyme solution were used in combination to prepare RD, RD content in the reaction solution is low. To further improve the yield of RD, GT708 was mutated in this example, and the mutation method comprised the following steps:

3.1. The primers shown in Table 4 were designed using the nucleotide sequence of GT708 as a template.

Table 4 Mutation primers for GT708

| Enzyme number | Primer number | Sequence | Sequence number |
|---|---|---|---|
| / | F9 | GTCGCGTTCTGAACAAACTGGTGCCGCAGGCCC | SEQ ID NO: 5 |
| / | R9 | GGCACCAGTTTGTTCAGAACGCGACCACGATCA | SEQ ID NO: 6 |
| GT828 | F10 | CGCGAGATGTTGGGAACATTTCCGCCTGAAGAAGATTTTT | SEQ ID NO: 7 |
| | R10 | TGTTCCCAACATCTCGCGCAACTTATCCTGTTCTCGCTTCG | SEQ ID NO: 8 |
| GT829 | F11 | TTCCGTTTCAGGCCCCTATCATGCTGATGTGCACG | SEQ ID NO: 9 |
| | R11 | TAGGGGCCTGAAACGGAACTAAAAAATCTTCTTCAGGCGG | SEQ ID NO: 10 |
| GT830 | F12 | GCGAAACTGATGGTGGAATTAGGCGTGGCAG | SEQ ID NO: 11 |
| | R12 | ATTCCACCATCAGTTTCGCGTTAATCGGCTGATC | SEQ ID NO: 12 |

(continued)

| Enzyme number | Primer number | Sequence | Sequence number |
|---|---|---|---|
| GT831 | F13 | GAACAAATTCGCGCCGCAGGCCCATATTCTG | SEQ ID NO: 13 |
| | R13 | CTGCGGCGCGAATTTGTTCAGAACGCGACC | SEQ ID NO: 14 |

In Table 4 above, F represents the forward primer and R represents the reverse primer . The numbers following F and R are the primer numbers used to distinguish between different primers. The enzyme numbers indicates the number of the mutant enzymes obtained after the mutation.

3.2. PCR amplification was performed using F10-13 / R9 and F9 / R10-13 as primers, and two fragments were obtained for each pair of primers. The two-fragment homologous recombinase provided by Vazyme Biotech Co.,Ltd. was used for recombination, and the recombinant products were transformed to *E. coli* Trans10 competent cells. The transformed *E. coli* Trans10 competent cells were spread onto LB medium with corresponding antibiotics and incubated upside down at 37°C overnight. Single colonies were picked and inoculated into test tubes with LB liquid medium (Km added), cultured for 8 to 10 h, and plasmids were extracted for sequencing identification. The sequencing results are shown in Table 5 below.

Table 5 Sequencing results of four enzyme mutants based on GT708

| Enzyme number | Recombinant plasmid number | Genetically engineered strain number | Mutation site as compared to the amino acid sequence of GT708 | Nucleotide sequence number | Amino acid sequence number |
|---|---|---|---|---|---|
| GT708 | pET28a GT708- | RMIS708 | / | SEQ ID NO: 3 | SEQ ID NO: 4 |
| GT828 | pET28a GT828- | RMIS828 | D323N-H324K-M181L-F185L-E188F | SEQ ID NO: 15 | SEQ ID NO: 16 |
| GT829 | pET28a GT829- | RMIS829 | D323N-H324K-A196V-A198F-G201P | SEQ ID NO: 17 | SEQ ID NO: 18 |
| GT830 | pET28a GT830- | RMIS830 | D323N-H324K-R373K-L375M | SEQ ID NO: 19 | SEQ ID NO:20 |
| GT831 | pET28a GT831- | RMIS831 | D323N-H324K-L325F-V326A | SEQ ID NO: 21 | SEQ ID NO: 22 |

Table 5 shows the mutation sites of the four enzyme mutants compared to the amino acid sequence of GT708. Among them, D323N means that the amino acid residue at position 323 of the amino acid sequence of GT828 is changed from D (aspartic acid) to N (asparagine) compared to that of GT708, and other mutation sites are described in the same way. The hyphen "-" is a separator that separates different mutation sites.

3.3 The four correctly sequenced plasmids were respectively transformed into host *E. coli* BL21 (DE3) competent cells to obtain four genetically engineered strains containing the point mutations shown in Table 5. Single colonies of the genetically engineered strains were respectively picked and inoculated into 5 mL LB liquid medium containing 50 μg/mL kanamycin and shaking cultured at 37°C for 4 h. Then the culture was transferred to 50 mL of freshly prepared TB liquid medium containing 50 μg/mL kanamycin at an inoculum amount of 2v/v% (volume percentage) and shaking cultured at 37°C until $OD_{600}$ reached about 0.8. IPTG was added to a final concentration of 0.1 mM, and the culture was induced at 25°C for 20 h. After the culture is completed, the culture broth was centrifuged at 4000 rpm for 20 min, then the supernatant was discarded and the precipitate was collected to obtain wet cells. The wet cells were stored at -20°C for future use.

3.4. The collected wet cells were suspended in PBS (50 mM, pH 6.0) at a ratio of 1:5 g / mL (mass / volume ratio, namely

m / v) to obtain four suspensions. Then, the four suspensions were homogenized (550 Mbar for 1.5 min) to obtain four homogenized enzyme solutions. The four homogenized enzyme solutions were centrifuged at 12,000 rpm for 2 min, then the precipitations were discarded, and the supernatants were collected to obtain four crude enzyme solutions, namely GT828 crude enzyme solution, GT829 crude enzyme solution, GT830 crude enzyme solution and GT831 crude enzyme solution.

3.5. The above four crude enzyme solutions were respectively stand at a constant temperature of 80°C for 15min to obtain GT828 reaction enzyme solution, GT829 reaction enzyme solution, GT830 reaction enzyme solution and GT831 reaction enzyme solution. All four reaction enzyme solutions contain β-1,2-glucosyltransferase.

**Example 4: Preparation of rebaudioside D using four reaction enzyme solutions and GT609 reaction enzyme solution**

In this example, the reaction systems for the preparation of rebaudioside D were respectively constructed using the four reaction enzyme solutions (containing β-1,2-GT) associated with GT708 and GT609 reaction enzyme solution (including SUS). The content of each component in these four reaction systems is shown in Table 6.

Table 6 Composition of the reaction system for rebaudioside D preparation

| Component | Concentration of mother liquor | Addition amount | Final concentration |
|---|---|---|---|
| GT609 reaction enzyme solution | Stock solution | 30 μL | / |
| GT828, GT829, GT 829, GT830 or GT831 reaction enzyme solution | Stock solution | 150 μL | / |
| Sucrose | 400 g/L | 500 μL in total | 200 g/L |
| Rebaudioside A | 100 g/L | | 50 g/L |
| NDP (ADP or UDP) | 10 g/L | 10 μL | 0.1 g/L |
| PBS buffer | 50 mM, pH 6.0 | allowance | / |
| Total volume of the reaction system | 1 mL | | |

In Table 6, the mother liquor concentration refers to the initial concentration of each component at the beginning of addition. Stock solution refers to each reaction enzyme solution was added in an undiluted state. The final concentration refers to the initial concentration of each component in the whole reaction system after addition, which is the concentration at the reaction time of 0h. NDP (ADP or UDP) means that the reaction system using ADP and the reaction system using UDP were prepared using ADP and UDP, respectively. Based on the four types of reaction enzyme solutions GT828, GT829, GT830 and GT831, as well as the two types of NDP, Table 6 includes eight reaction systems.

The preparation method of rebaudioside D (RD) in this example comprises the following steps:

Eight reaction systems were prepared according to Table 6. Each reaction system was respectively placed in a metal bath (at a set heating temperature of 60°C) and reacted at 60°C for 30 min to obtain eight reaction solutions. The eight reaction solutions were respectively diluted by 50-fold and sampled for HPLC analysis. Meanwhile, the reaction system containing GT708 was set as a control. The results of the HPLC analysis are shown in Table 7.

Table 7 HPLC results of the reaction solutions obtained from different reaction systems containing β-1,2-GT

| Enzyme number | Genetically engineered strain number | Reaction systems using ADP | | Reaction systems using UDP | |
|---|---|---|---|---|---|
| | | RD concentration (g/L) | RD yield | RD concentration (g/L) | RD yield |
| GT708 | RMIS708 | 38.95 | 66.57% | 16.83 | 28.77% |
| GT828 | RMIS828 | 41.53 | 70.99% | 27.15 | 46.41% |
| GT829 | RMIS829 | 55.23 | 94.40% | 28.92 | 49.44% |
| GT830 | RMIS830 | 48.47 | 82.86% | 28.73 | 49.11% |
| GT831 | RMIS831 | 39.76 | 67.96% | 27.57 | 47.13% |

In Table 7, the concentration of RD refers to the ratio (g/L) of the mass (g) of RD contained in the reaction solution to the volume (L) of the reaction solution after the termination of the reaction. RD yield (ADP) represents the yield of RD at the end of the reaction when the reaction system uses ADP (without UDP). RD yield (UDP) represents the yield of RD at the end of the reaction when the reaction system uses UDP.

As shown in Table 7, RD yield of the reaction system using ADP is higher than that of the reaction system with UDP. In addition, for GT829, the RD yield is significantly higher than that of other enzymes (GT828, GT830, GT831) in both the reaction system using ADP and the reaction system using UDP, so GT829 was selected for the preparation of RM.

**Example 5: Preparation of GT109 reaction enzyme solution**

The present example relates to the preparation method of the GT109 reaction enzyme solution. GT109 belongs to β-1,3-glucosyltransferase (abbreviated as β-1,3 GT). β-1,3-glucosyltransferase can use rebaudioside D as the substrate, and transfer the glucosyl group from the glycosyl donor to the sugar group at position C-19 of rebaudioside D to generate rebaudioside M (RM). The glycosyl donor can be NDP-glucose, including ADP-glucose, UDP-glucose, CDP-glucose, GDP-glucose, TDP-glucose, and IDP-glucose, etc.

The GT109 of this example is Enzyme 24 (Enz.24) as described in paragraph 0130 of the specification of the invention patent application with the publication number CN115418358A. The nucleotide sequence of GT109 is shown as SEQ ID NO: 23, and the amino acid sequence of GT109 is shown as SEQ ID NO: 24.

The preparation method of the GT109 reaction enzyme solution provided in this example was the same as those described in Example 1. The difference was that the recombinant plasmid obtained was pET28a-GT109. The resulting genetically engineered strain containing the recombinant plasmid pET28a-GT109 was designated as RMIS109.

In this example, the homogenization ratio of GT109 wet cells used to produce GT109 crude enzyme solution was 1 g: 5 mL.

**Example 6: Preparation of rebaudioside M using GT109 reaction enzyme solution, GT829 reaction enzyme solution and GT609 reaction enzyme solution**

In this example, Rebaudioside M was prepared using GT109 reaction enzyme solution (containing β-1,3-glucosyltransferase), GT829 reaction enzyme solution (containing β-1,2-glucosyltransferase) and GT609 reaction enzyme solution (containing sucrose synthase). GT109 reaction enzyme solution was obtained from Example 5, GT829 reaction enzyme solution was obtained from Example 3, and GT609 reaction enzyme solution was obtained from Example 1.

A reaction system for preparing rebaudioside M was constructed in this example. The content of each component in this reaction system is shown in Table 8.

Table 8 Compositions of the reaction system for rebaudioside M preparation

| Component | Concentration of mother liquor | Addition amount | Final concentration |
|---|---|---|---|
| GT609 reaction enzyme solution | Stock solution | 60 μL | / |
| GT829 reaction enzyme solution | Stock solution | 120 μL | / |
| GT109 reaction enzyme solution | Stock solution | 150 μL | / |
| Sucrose | 400 g/L | 500 μL in total | 200 g/L |
| Rebaudioside A (RA) | 200 g/L | | 100 g/L |
| NDP (ADP or UDP) | 10 g/L | 10 μL | 0.1 g/L |
| PBS buffer | 50 mM, pH 6.0 | allowance | / |
| Total volume of the reaction system | 1 mL | | |

The reaction mechanism of the reaction system shown in Table 8 is as follows. Sucrose and NDP are converted into NDP-glucose (ADP-glucose or UDP-glucose) and D-fructose under the action of sucrose synthase (SUS) in GT609 reaction enzyme solution. Rebaudioside A (RA) and NDP-glucose (as a glycosyl donor) are converted into rebaudioside D (RD) under the action of β-1,2-glucosyltransferase (β-1,2-GT) in GT829 reaction enzyme solution. Rebaudioside D (RD) and NDP-glucose (as a glycosyl donor) are converted into rebaudioside M (RM) under the action of β-1,3-glucosyltransferase (β-1,3-GT) in the GT109 reaction enzyme solution.

In Table 8, NDP (ADP or UDP) means that the reaction system using ADP and the reaction system using UDP were respectively prepared, so Table 8 includes two reaction systems.

The preparation method of rebaudioside M in this example comprises the following steps:
Two reaction systems were prepared according to Table 8. Each reaction system was respectively placed in a metal bath (at a set heating temperature of 60°C) and reacted at 60°C for 6h or 22h. After the termination of the reaction, two reaction solutions were obtained. The two reaction solutions were respectively diluted by 50-fold and sampled for HPLC analysis. The results of the HPLC analysis are shown in Table 9.

Table 9 Content of each product in the reaction solutions

| Reaction system | Reaction time | RA | | RD | | RM | | |
|---|---|---|---|---|---|---|---|---|
| | | Concentration (g/L) | Percent content | Concentration (g/L) | Percent content | Concentration (g/L) | Percent content | Yield |
| The reaction system using ADP | 6 h | 1.17 | 0.88% | 4.75 | 3.57% | 126.99 | 95.55% | 94.77% |
| The reaction system using UDP | 6 h | 7.49 | 5.77% | 12.13 | 9.35% | 110.08 | 84.87% | 82.15% |
| | 22 h | 0.48 | 0.36% | 9.83 | 7.42% | 122.10 | 92.22% | 92.12% |

In Table 9, the reaction system using ADP means that one of the substrates of the sucrose synthase in the reaction system is ADP (not UDP), so the glycosyl donor is ADP-glucose. The reaction system using UDP means that one of the substrates of the sucrose synthase in the reaction system is UDP (not ADP), so the glycosyl donor is UDP-glucose. The concentration of RA refers to the ratio (g/L) of the mass (g) of RA contained in the enzyme-catalyzed final product system to the volume (L) of the enzyme-catalyzed final product system after the termination of the reaction. The same is applied for the concentrations of RD and RM. The percentage content of RA refers to the percentage of the mass of RA relative to total mass of RA, RD and RM contained in the enzyme-catalyzed final product system obtained after the termination of the reaction. The same is applied for the percentage content of RD and RM.

According to the results in Table 9, in the reaction system using ADP, the yield of RM reached 94.77% after 6h of reaction. However, in the reaction system using UDP, the yield of RM reached 91.12% after 22h of reaction, but it was still lower than the yield of RM achieved in the reaction system using ADP after 6h of reaction. This indicates that for GT829, the yield of RM was higher with ADP-glucose as glycosyl donor than with UDP-glucose as glycosyl donor.

The addition amounts of each example of the present invention are shown in Table 10.

Table 10 Addition amounts in each example of the present invention

| Example | Type of enzyme | Added volume of reaction enzyme solution (mL) | Mass of wet cells (g) | Add concentration of wet cells (g/L) | Added concentration of RA (g/L) | Added mass of RA (g) | Add volume of crude enzyme solution : added mass of RA (mL/g) | Ratio of the added mass of wet cells to RA |
|---|---|---|---|---|---|---|---|---|
| Example 2 | Sucrose synthase GT609 | 0.03 | 0.006 | 6 | 50 | 0.05 | 0.6:1 | 0.12:1 |
| | β-1,2-glucosyltransferase GT708 | 0.15 | 0.03 | 30 | 50 | 0.05 | 3:1 | 0.6:1 |

(continued)

| Example | Type of enzyme | Added volume of reaction enzyme solution (mL) | Mass of wet cells (g) | Add concent ration of wet cells (g/L) | Added concent ration of RA (g/L) | Added mass of RA (g) | Add volume of crude enzyme solution : added mass of RA (mL/g) | Ratio of the added mass of wet cells to RA |
|---|---|---|---|---|---|---|---|---|
| Example 4 | Sucrose synthase GT609 | 0.03 | 0.006 | 6 | 50 | 0.05 | 0.6:1 | 0.12:1 |
| | β-1,2-glucosyltransferase GT828 | 0.15 | 0.03 | 30 | 50 | 0.05 | 3:1 | 0.6:1 |
| | β-1,2-glucosyltransferase GT829 | 0.15 | 0.03 | 30 | 50 | 0.05 | 3:1 | 0.6:1 |
| | β-1,2-glucosyltransferase GT830 | 0.15 | 0.03 | 30 | 50 | 0.05 | 3:1 | 0.6:1 |
| | β-1,2-glucosyltransferase GT831 | 0.15 | 0.03 | 30 | 50 | 0.05 | 3:1 | 0.6:1 |
| Example 6 | Sucrose synthase GT609 | 0.06 | 0.012 | 12 | 100 | 0.1 | 0.6:1 | 0.12:1 |
| | β-1,2-glucosyltransferase GT829 | 0.12 | 0.024 | 24 | 100 | 0.1 | 1.2:1 | 0.24:1 |
| | β-1,3-glucosyltransferase GT109 | 0.15 | 0.03 | 30 | 100 | 0.1 | 1.5:1 | 0.3:1 |

**Example 7: Preparation of RebM via enzyme-catalyzed amplification reaction using RA97 as raw material**

11.3g of β-1,3-glucosyltransferase GT109 bacterial sludge (produced by 5L-scale fermentation) was homogenized with PBS buffer at pH 7.0 to prepare 50 g of enzyme solution, 7.7 g of sucrose synthase GT609 bacterial sludge (produced by 5L-scale fermentation) was homogenized with PBS buffer at pH 7.0 to prepare 80 g of enzyme solution; 6.4g of β-1,2-glucosyltransferase GT829 bacterial sludge (produced by 5L-scale fermentation) was homogenized with PBS buffer at pH 7.0 to prepare 61 g of enzyme solution.

200 g of water was added to a 500 mL reactor, then stir was started and the water was heated to 60°C. 15 g of RA97 and 26 g of sucrose was added respectively, and stirred until completely dissolved, maintaining a temperature above 58°C during dissolution. Subsequently, 2.7 g of GT109 enzyme solution, 1.5 g of GT609 enzyme solution and 1.5 g of GT829 enzyme solution, and 0.075g of ADP were added. The pH was adjusted to 6.0-6.2 using 25% dilute phosphoric acid, in an addition amount of about 1.5g, and then the reaction was started.

The distribution of steviol glycoside components was detected by HPLC (JECFA, 2021) during the reaction. The product began to precipitate after about 1.5 hours of reaction.

Multiple batches of reactions were performed in the present invention, among which two batches (SG00120240510, SG00120240511) obtained the following data in Table 11 respectively. Specifically, in batch SG00120240510, after 1 hour of reaction, the distribution of RebA and stevioside decreased by 91.9% and the distribution of Reb D and Reb M increased by 90.45%; after 2 hours of reaction, the distribution of RebA and stevioside decreased by 97.7% and the distribution of Reb D and Reb M increased by 97.45%.

The data indicates that with the passage of enzyme reaction time, the main conversion from RebA to Reb D and Reb M occured within the first 2 hours of the reaction time. FIG. 2 shows that RebA decreased from 98% to less than 1% after 2 hours, and Reb D first increased to more than 25% within 1 hour and decreased to 2% after 2 hours. Thus, after 2 hours of reaction, the distribution of Reb M increased to more than 95%, and the distribution of Reb D and Reb M increased to more than 97%. Data on the distribution change of steviol glycosides are shown in Table 11.

Table 11 Distribution of steviol glycosides at different times of biotransformation

| Steviol Glycoside (%) | Bioconversion Time (hours) | | | | | | | | |
|---|---|---|---|---|---|---|---|---|---|
| | 0 | 1 | 2 | 3 | 4 | 5 | 6 | 7 | 8 |
| Batch SG00120240510 | | | | | | | | | |
| Rebaudioside E | ND | 1.635 | 0.429 | 0.448 | 0.420 | 0.415 | 0.411 | 0.432 | 0.411 |
| Rebaudioside D | 0.444 | 24.418 | 2.384 | 2.224 | 2.304 | 2.070 | 2.229 | 2.048 | 2.243 |
| Rebaudioside M | ND | 66.484 | 95.512 | 95.764 | 95.755 | 95.975 | 95.867 | 95.994 | 95.846 |
| Rebaudioside I | 0.170 | 0.456 | 0.504 | 0.469 | 0.452 | 0.453 | 0.440 | 0.443 | 0.426 |
| Rebaudioside A | 98.101 | 6.301 | 0.496 | 0.482 | 0.467 | 0.469 | 0.464 | 0.466 | 0.455 |
| Stevioside | 0.146 | 0.072 | ND | ND | ND | ND | ND | ND | ND |
| Rebaudioside F | 0.369 | ND | ND | ND | ND | ND | ND | ND | ND |
| Rebaudioside C | 0.160 | ND | ND | ND | ND | ND | ND | ND | ND |
| Rebaudioside B | 0.609 | 0.634 | 0.675 | 0.614 | 0.602 | 0.619 | 0.589 | 0.617 | 0.619 |
| Total Steviol Glycosides(%) | 100.00 | 100.00 | 100.00 | 100.00 | 100.00 | 100.00 | 100.00 | 100.00 | 100.00 |
| | | | | | | | | | |
| Batch SG00120240511 | | | | | | | | | |
| Rebaudioside E | ND | 1.174 | 1.235 | 0.486 | 0.475 | 0.464 | 0.473 | 0.464 | 0.465 |
| Rebaudioside D | 0.579 | 31.712 | 5.200 | 2.047 | 1.932 | 1.893 | 2.001 | 1.871 | 1.860 |
| Rebaudioside M | ND | 47.454 | 91.458 | 95.992 | 96.088 | 96.184 | 96.083 | 96.229 | 96.180 |
| Rebaudioside I | 0.172 | 0.424 | 0.467 | 0.426 | 0.429 | 0.408 | 0.404 | 0.394 | 0.392 |
| Rebaudioside A | 97.962 | 18.313 | 0.999 | 0.420 | 0.431 | 0.435 | 0.420 | 0.423 | 0.429 |
| Stevioside | 0.151 | 0.205 | ND | ND | ND | ND | ND | ND | ND |
| Rebaudioside F | 0.367 | 0.058 | ND | ND | ND | ND | ND | ND | ND |
| Rebaudioside C | 0.154 | 0.058 | ND | ND | ND | ND | ND | ND | ND |
| Rebaudioside B | 0.615 | 0.602 | 0.642 | 0.629 | 0.645 | 0.616 | 0.619 | 0.618 | 0.674 |
| Total Steviol Glycosides(%) | 100.00 | 100.00 | 100.00 | 100.00 | 100.00 | 100.00 | 100.00 | 100.00 | 100.00 |

**Example 8: Catalytic preparation of Reb D using RA97 as raw material**

100 g of substrate RA97, 50 g of sucrose and 0.9 L of water were added into a 2 L three-mouth flask, then stirred to dissolve the materials and heated the mixture to 60°C. After the materials are completely dissolved, β-1,2-glucosyltransferase GT829 enzyme solution (6 g of bacterial sludge produced by 5L-scale fermentation homogenized with 24 mL of PBS buffer at pH7.0), sucrose synthase GT609 enzyme solution (6g of bacterial sludge produced by 5L-scale fermentation homogenized with 24mL of PBS buffer at pH7.0) and 0.3 g of ADP were added. The total reaction volume was around 1 L-1.2 L. The pH was adjusted to 6.0-6.2 using 25% dilute phosphoric acid, then the reaction was started at a controlled temperature of 60°C.

The distribution of steviol glycoside components was detected by HPLC (JECFA, 2021) during the reaction.

Multiple batches of reactions were performed in the present invention, among which two batches (A319-156-1, A319-158-1) obtained the following data in Table 12 respectively. Taking A319-156-1 as an example, after 1 h of reaction, the distribution of RebA decreased by 74.23%, and the distribution of Reb D increased by 74.80%; after 2 h of reaction, the distribution of RebA decreased by 86.13%, and the distribution of Reb D and Reb M increased by 86.68%. Data on the distribution change of steviol glycosides are shown in Table 12.

Table 12 Distribution of steviol glycosides at different times of biotransformation

| Steviol Glycoside(%) | Time (hours) | | | | | | | | |
|---|---|---|---|---|---|---|---|---|---|
| | 0 | 0.5 | 1 | 2 | 3 | 4 | 5 | 6 | 21 |
| Batch No.A319-156-1 | | | | | | | | | |
| Rebaudioside E | 0.748 | 0.861 | 0.672 | 0.836 | 1.131 | 1.208 | 1.002 | 0.854 | 1.25 |
| Rebaudioside D | 1.284 | 50.923 | 76.087 | 87.964 | 88.021 | 88.072 | 88.414 | 88.645 | 89.074 |
| Rebaudioside I | 0.189 | 0.143 | 0.141 | 0.115 | 0.141 | 0.133 | 0.130 | 0.134 | 0.115 |
| Rebaudioside A | 96.566 | 47.177 | 22.338 | 10.439 | 10.116 | 10.021 | 9.914 | 9.814 | 9.097 |
| Rebaudioside F | 0.351 | 0.170 | 0.106 | 0.064 | ND | ND | ND | ND | ND |
| Rebaudioside C | 0.319 | 0.210 | 0.139 | 0.074 | ND | ND | ND | ND | ND |
| Rebaudioside B | 0.543 | 0.516 | 0.516 | 0.509 | 0.592 | 0.566 | 0.54 | 0.552 | 0.464 |
| Total Steviol Glycosides(%) | 100.00 | 100.00 | 100.00 | 100.00 | 100.00 | 100.00 | 100.00 | 100.00 | 100.00 |
| | | | | | | | | | |
| Steviol Glycoside(%) | Time (hours) | | | | | | | | |
| | 0 | 0.5 | 1 | 2 | 3 | 4 | 5 | 6 | 22 |
| Batch No.A319-158-1 | | | | | | | | | |
| Rebaudioside E | 0.367 | 0.424 | 0.488 | 0.305 | 0.511 | 0.383 | 0.906 | 0.758 | 0.582 |
| Rebaudioside D | 1.396 | 46.656 | 69.416 | 89.400 | 89.510 | 89.842 | 89.378 | 89.619 | 89.347 |
| Rebaudioside I | 0.185 | 0.176 | 0.169 | 0.124 | 0.125 | 0.118 | 0.144 | 0.124 | 0.104 |
| Rebaudioside A | 96.947 | 51.859 | 29.242 | 9.659 | 9.383 | 9.176 | 8.982 | 8.942 | 9.529 |
| Rebaudioside F | 0.352 | 0.238 | 0.101 | ND | ND | ND | ND | ND | ND |
| Rebaudioside C | 0.296 | 0.191 | 0.135 | ND | ND | ND | ND | ND | ND |
| Rebaudioside B | 0.458 | 0.456 | 0.449 | 0.512 | 0.471 | 0.48 | 0.59 | 0.557 | 0.492 |
| Total Steviol Glycosides(%) | 100.00 | 100.00 | 100.00 | 100.00 | 100.00 | 100.00 | 100.00 | 100.00 | 100.05 |

**Example 9: Catalytic preparation of Reb M using RA60 as raw material**

60 mL of water was added into a 250mL three-mouth flask and the temperature was controlled at 60°C. 10g of RA60 was added under stirring, then 20g of sucrose was added. After the materials are completely dissolved, 0.3 g of ADP was added. Subsequently, the pH was adjusted to 6.0-6.2 using 10% sodium hydroxide solution. After that, β-1,2-glucosyltransferase GT829 enzyme solution (3g of bacterial sludge produced by 5L-scale fermentation homogenized with 15mL of PBS buffer at pH7.0), β-1,3-gluccosyltransferase GT109 enzyme solution (6g of bacterial sludge produced by 5L-scale fermentation homogenized with 30mL of PBS buffer at pH7.0) and sucrose synthase GT609 enzyme solution (3g of bacterial sludge produced by 5L-scale fermentation homogenized with 15 mL of PBS buffer at pH7.0) were added. The pH was adjusted to 6.0-6.2 using 25% dilute phosphoric acid and the reaction was started.

The distribution of steviol glycoside components was detected by HPLC (JECFA, 2021) during the reaction. The results are shown in Table 13. After 1 h of reaction, the distribution of RebA and stevioside decreased by 39.77%, and the distribution of Reb D and Reb M reached 41.57%, with an increase of 37.64%. After 2 h of reaction, the distribution of RebA and stevioside decreased by more than 63.54%, and the distribution of Reb D and Reb M increased by 62.98%. Data on the distribution change of steviol glycosides are shown in Table 13.

Table 13 Distribution of stevia ol glycosides at different times of biotransformation

| Steviol Glycoside(%) | 0h | 0.5h | 1h | 1.5h | 2h | 2.5h | 3h | 3.5h | 4h | 5h | 6h |
|---|---|---|---|---|---|---|---|---|---|---|---|
| Rebaudioside E | 0.900 | 1.489 | 1.5 | 1.378 | 1.138 | 0.989 | 0.684 | 0.208 | 0.000 | 0.000 | 0.000 |

(continued)

| Steviol Glycoside(%) | 0h | 0.5h | 1h | 1.5h | 2h | 2.5h | 3h | 3.5h | 4h | 5h | 6h |
|---|---|---|---|---|---|---|---|---|---|---|---|
| Rebaudioside D | 2.062 | 5.627 | 8.233 | 9.369 | 9.512 | 8.980 | 7.683 | 3.449 | 2.013 | 1.489 | 1.464 |
| Rebaudioside N | 0.633 | 1.301 | 1.649 | 1.943 | 2.176 | 2.087 | 2.313 | 2.086 | 1.110 | 0.851 | 0.830 |
| Rebaudioside M | 1.859 | 16.595 | 33.333 | 44.503 | 57.389 | 64.933 | 73.198 | 84.551 | 90.420 | 92.254 | 92.571 |
| Rebaudioside I | 0.384 | 1.078 | 1.725 | 2.043 | 2.502 | 2.702 | 2.886 | 3.285 | 3.309 | 3.345 | 3.225 |
| Rebaudioside A | 64.143 | 52.21 | 36.959 | 27.088 | 16.940 | 11.881 | 6.311 | 2.589 | 0.858 | 0.695 | 0.675 |
| Stevioside | 18.425 | 9.875 | 5.836 | 3.864 | 2.090 | 1.278 | 0.533 | 0.220 | 0.050 | 0.000 | 0.000 |
| Rebaudioside F | 1.581 | 1.189 | 0.919 | 0.706 | 0.512 | 0.341 | 0.145 | 0.060 | 0.038 | 0.000 | 0.000 |
| Rebaudioside C | 9.293 | 8.575 | 7.749 | 7.023 | 5.905 | 5.104 | 3.683 | 2.151 | 0.664 | 0.029 | 0.026 |
| Rubusoside | 0.604 | 1.03 | 1.074 | 1.000 | 0.698 | 0.544 | 1.333 | 0.224 | 0.436 | 0.218 | 0.107 |
| Rebaudioside B | 0.874 | 0.894 | 0.901 | 0.956 | 1.027 | 1.070 | 1.045 | 1.121 | 1.1 | 1.119 | 1.101 |
| Steviolbioside | 0.142 | 0.139 | 0.121 | 0.125 | 0.112 | 0.092 | 0.186 | 0.059 | 0 | 0 | 0 |
| Total Steviol Glycosides(%) | 100.00 | 100.00 | 100.00 | 100.00 | 100.00 | 100.00 | 100.00 | 100.00 | 100.00 | 100.00 | 100.00 |

As can be seen from Examples 7, 8 and 9, by using this process, high distribution of Reb D, Reb M and their combination can be achieved in just 1 or 2 hours, making the process very efficient in industrial-scale production. The data in Examples 7,8 and 9 are summarized in the table below.

Table 14 Distribution changes of steviol glycosides after 1 h, 2 h and 4 h of biotransformation

| Raw material | Generate / consume | Change in 1h | Change in 2h | Change in 4h | Change in 1h | Change in 2h | Change in 4h |
|---|---|---|---|---|---|---|---|
| | Batch No | SG00120240510 | | | SG00120240511 | | |
| Reb A 97 | Reb M | 66.48 | 95.51 | 95.76 | 47.45 | 91.46 | 96.09 |
| | Reb D | 23.97 | 1.94 | 1.86 | 31.13 | 4.62 | 1.35 |
| | Reb M+Reb D | 90.45 | 97.45 | 97.62 | 78.58 | 96.08 | 97.44 |
| | Reb A | -91.80 | -97.61% | -97.63 | -79.65 | -96.96 | -97.53 |
| | | | | | | | |
| | Batch No | A319-156-1 | | | A319-158-1 | | |
| Reb A 97 | Reb D | 64.803 | 86.68 | 86.79 | 68.02 | 88.00 | 88.45 |
| | Reb A | -64.228 | -86.13 | -86.55 | -67.705 | -87.29 | -87.77 |
| | | | | | | | |

(continued)

| | Batch No | A319-171-c | | | | | |
|---|---|---|---|---|---|---|---|
| Stevia leaf extract: RA60 | Reb M | 31.47 | 55.53 | 88.56 | | | |
| | Reb D | 6.17 | 7.45 | -0.05 | | | |
| | Reb M+Reb D | 37.64 | 62.98 | 88.51 | | | |
| | Reb A | -27.18 | -47.20 | -63.29 | | | |
| | Stevioside | -12.59 | -16.34 | -18.38 | | | |
| | Reb A+Stev | -39.77 | -63.54 | -81.67 | | | |

Sequences used in the present invention

Nucleotide sequence of GT609 (SEQ ID NO: 1):

ATGTCCGAACGTGCGCGTAGCGATGATCTGATTGAACAGCTGCGCGGCTTCCTG
GATGCTTGTCCGTCCGTGGGTCATCGCGTTCTGCACCAGATTAAAAAACTGGAACGC
TCCTTCCTTCAGCGTTCTGAACTGTGCGACGCGTTTGCAGATGTTTGCGCAGCCGAA
GATGTTCCAGATGGCCTGCGCCAGAGCCCGCTGGGTAAAGTTATTCGTCTGACCCAG
GAAGCCGCTGTTAACGATGCTTGGGTTTACCTGGCTGTTCGTGTGCGTATTGCGTCCT
GGCGTTACGTTCGTATCGCGCTGGAAGGTATGGCGGTTGAAGAAGTGACTGTTCGTG
ATTTCCTGCGTTTCAAAGAATGTCTGGCTTTGGGCCAGCACGATTTTGATGAATGGCT
GCTGGAAATTGATCTGGGTCCGTTCTCTCGTGAATTTCCGAAACTGCTGGAAGCCCGT
TCCATTGGCCGTGGTGTTGAATTTCTGAACAAACATCTGAGCCTGCAACTGTTCGACG
AACTGGGTGAAGGTGGTGAACGCATCCTGAACTTCCTGCGCATCCACTCTTTTCGCG
GCCAGACCCTGATGCTGAACGAACAGATTAAATCCGTGTCTGATCTCCGTCGTGCACT
GCGTCGTGCGGATGAAGTGTTGGCCGGTCACGAGCCGACGGCGACCTGGGAAGATG
TGGCTGCACCGCTGCGCGCACTGGGTTTCGAAGTTGGTTGGGGTCGTGAAGTTGCGC
GTATCCGTGACACCATGAGCCTGCTGCGTGATCTGCTGGAAGCACCAGACCCTCGTG
GCCTGGAAACCTTTCTGGCTCGTCTGCCGATGGTGTTTTCTCTGGCTATCATTTCTCCG

CACGGTTACTTCGGTCAGGCTAACGTTCTGGGTCGTCCGGATACTGGTGGTCAGGTG
GTATACATTCTGGACCAGGTTCGTGCCCTCGAAACCGAGATGCGCTCTCGCCTGTTCG
AACAGGGCCTGGACATCGAACCGCAGATTGTTGTCCTGACCCGTCTGATCCCGCAGG
CTGAAGGCACCACCTGCGATCAGCGTCTGGAACCGATCTCTGGCACTCGCAACGCGC
GCATCCTGCGTGTCCCGTTCCGTAACGCCAGCGGTGAAATCGTTACCCACTGGATTTC
TCGTTTCGAGGTGTGGCCTTACCTGGAACGTTACACTCTGGATAGCGAACGTGAACT
GCTGGCTGAACTGGGTGGCCGTCCGGATCTGATCGTTGGTAATTATAGCGACGGTAAC
CTGGTGGCTACCCTGCTGTCCCAGCGCCTGGGCGTGACCCAGTGCAACATCGCACAC
GCGCTGGAAAAAACCAAATACCCTCACGCCGACCTGTTCTGGCAGGAAAACGAAGC
GCAGTATCATTTTAGCTGCCAGTTTACCGCGGATCTGATTGCTATGAACGCTGCTGACT
TTATCATCACGTCTACCTACCAGGAAATCGCTGGTACGCGCGAATCCGTTGGCCAGTA
TGAGAGCCACACCGCGTTCACCATGCCGAAACTGTTCCGTGTGGTCAACGGCATTGA
CGTGTATGACCCAAAATTCAACATCGTGAGTCCGGGCGCCGACGCAGCTGCATACTTC
CCGTATACCGCGGTGGAACGTCGCCTCCCGCATCTGCACACTGAAATCGAACAGCTG
GTTTTCGGCGTGGATGAACGTGTTGACGCGCGCGGCGTGCTGACCGAACGTGACAA
ACCGCTGCTGTTCACTATGGCTCGTTTGGACCGTATCAAAAACATCGTTGGTCTGGTT
GAATGGTTCGGCGCGTGCGAAGCGCTGCGTAAAGAAGCGAATCTGCTGGTCATCTCA
GGTCACGTAGACCCGGAACGCTCATCCGACACTGAAGAACTGGAACAGATCCGTTGT
ATGCACGCTCTGTTCAACCGTTATGATCTGGATCGTCAGGTGCGCTGGCTGGGTCTGC
GTCTGCCGAAAGATCTGGCGGGTGAATTTTACCGCTATGTTGCGGACGGTCGTGGCG
CGTTCGTTCAGCCGGCTCTGTTTGAAGCATTCGGTCTGACCGTGATTGAAGCGATGGC
GTCTGGCCTGCCGTGCTTCGCAACCTGCTTCGGCGGCCCGTCTGAAATCATTGAAGAT
GGTGTCTCTGGCTTCCACATCGATCCGAACCACGGCGATGCGGCAGCGGAACGTATC
GCGCGCTTTTTCGAACGTACCCGTCAAGATCCGGAATATTGGAACCGTATCTCTGAAG
GCGCGCTGAAACGTGTGGCCGAACGCTACACCTGGCAGCATTATGCTGAACGTATGA
TGACCCTGAGCCGTGTTTACGGCTTCTGGCGTCATGTTACTGACCTGGAACGTCGTGA
AACCCAGCGTTACCTGCAAGCTCTGTATTCTCTGCAATTCCGTCGTCTGGCGCAGGCG
ATGGCG

Amino acid sequence of GT609 (SEQ ID NO: 2):

MSERARSDDLIEQLRGFLDACPSVGHRVLHQIKKLERSFLQRSELCDAFADVCAAE
DVPDGLRQSPLGKVIRLTQEAAVNDAWVYLAVRVRIASWRYVRIALEGMAVEEVTVRDF
LRFKECLALGQHDFDEWLLEIDLGPFSREFPKLLEARSIGRGVEFLNKHLSLQLFDELGE

GGERILNFLRIHSFRGQTLMLNEQIKSVSDLRRALRRADEVLAGHEPTATWEDVAAPLRA
LGFEVGWGREVARIRDTMSLLRDLLEAPDPRGLETFLARLPMVFSLAIISPHGYFGQANV
LGRPDTGGQVVYILDQVRALETEMRSRLFEQGLDIEPQIVVLTRLIPQAEGTTCDQRLEPI
SGTRNARILRVPFRNASGEIVTHWISRFEVWPYLERYTLDSERELLAELGGRPDLIVGNYS
DGNLVATLLSQRLGVTQCNIAHALEKTKYPHADLFWQENEAQYHFSCQFTADLIAMNA
ADFIITSTYQEIAGTRESVGQYESHTAFTMPKLFRVVNGIDVYDPKFNIVSPGADAAAYFP
YTAVERRLPHLHTEIEQLVFGVDERVDARGVLTERDKPLLFTMARLDRIKNIVGLVEWFG
ACEALRKEANLLVISGHVDPERSSDTEELEQIRCMHALFNRYDLDRQVRWLGLRLPKDL
AGEFYRYVADGRGAFVQPALFEAFGLTVIEAMASGLPCFATCFGGPSEIIEDGVSGFHIDP
NHGDAAAERIARFFERTRQDPEYWNRISEGALKRVAERYTWQHYAERMMTLSRVYGFW
RHVTDLERRETQRYLQALYSLQFRRLAQAMA

Nucleotide sequence of GT708 (SEQ ID NO: 3):

ATGCACCATCATCATGAAGGCGTGAGCGACCAGACCCTGAGAGTAACGATGTTT
CCGTGGCTTGGGCTGGGTCATGTTAACCCGTTTTTGCGTATCGCTAAACAACTGGCCG
ATCGTGGTTTCGTTATCTATTTAGTTAGTACCGCTATTAACCTCGAAATGATCAAAAAG
AGAATCCCGGAGAAATACAGTAATAGCATCCATCTGGTTGAGCTGCGCCTGCCAGAAT
TACCGGAACTGCCACCACATTACCATACTACCAACGGTTTACCACCGCATCTGAACAA
AACCCTGCACAAGGCACTGAAGATGAGCGCTCCCAACTTTAGCAAGATCCTTCAAAA
TATTAAGCCGGACCTGGTCCTTTACGATTTTCTGGTTCCGTGGGCAGAAAAAGTCGCG
CTTGAACAGGGCATCCCGGCTGTTCCATTGCTAACCAGTGGTGCGGCACTGTTCAGC
TACTTTTTCAACTTCCTGAAGCGACCGGGTGAAGAGTTTCCGTTTGAGGCAATCCGCC
TGTCGAAGCGAGAACAGGATAAGATGCGCGAGATGTTTGGAACAGAGCCGCCTGAA
GAAGATTTTTTAGCGCCGGCCCAGGCCGGTATCATGCTGATGTGCACGAGCCGCGTAA
TTGAGGCTAAGTACCTGGACTATTGTACCGAACTGACCAATGTAAAAGTTGTTCCGGT
TGGTCCGCCGTTTCAGGATCCGCTGACCGAAGATATTGACGACCCCGAACTGATGGAT
TGGTTAGATACCAAACCCGAACATAGTGTTGTCTATGTGTCGTTTGGCAGCGAAGCGT
TCCTGAGCCGTGAAGATATGGAAGAAGTCGCGTTCGGCCTGGAGCTGAGCGGCGTG
AACTTTATCTGGGTTGCACGCTTTCCGAAAGGCGAAGAACAGCGTCTGGAAGACGTT
CTGCCAAAAGGCTTCCTGGAACGCGTTGGTGATCGTGGTCGCGTTCTGGACCATCTG
GTGCCGCAGGCCCATATTCTGAACCATCCGAGCACGGGTGGCTTCATCTCTCATTGCG
GTTGGAACAGCGTCATGGAAAGCATTGATTTCGGCGTTCCGATCATTGCGATGCCGAT
GCAGTGGGATCAGCCGATTAACGCGAGACTGCTTGTGGAATTAGGCGTGGCAGTGGA

GATCCCGCGTGATGAAGATGGCCGGGTCCACCGCGCCGAAATTGCCGAAGCACTGAA AAGCGTTGTGACCGGTGAAACCGGCGAAATCCTGCGTGCGAAAGTTCGTGAAATCA GCAAAAACCTGAAATCCATCCGTGACGAAGAAATGGACGCGGTTGCTGAAGAACTG ATCCAGCTGTGCCGTAACTCTAACAAAAGCAAA

Amino acid sequence of GT708 (SEQ ID NO: 4):

MHHHHEGVSDQTLRVTMFPWLGLGHVNPFLRIAKQLADRGFVIYLVSTAINLEMIK KRIPEKYSNSIHLVELRLPELPELPPHYHTTNGLPPHLNKTLHKALKMSAPNFSKILQNIKP DLVLYDFLVPWAEKVALEQGIPAVPLLTSGAALFSYFFNFLKRPGEEFPFEAIRLSKREQDK MREMFGTEPPEEDFLAPAQAGIMLMCTSRVIEAKYLDYCTELTNVKVVPVGPPFQDPLTE DIDDPELMDWLDTKPEHSVVYVSFGSEAFLSREDMEEVAFGLELSGVNFIWVARFPKGE EQRLEDVLPKGFLERVGDRGRVLDHLVPQAHILNHPSTGGFISHCGWNSVMESIDFGVPII AMPMQWDQPINARLLVELGVAVEIPRDEDGRVHRAEIAEALKSVVTGETGEILRAKVREI SKNLKSIRDEEMDAVAEELIQLCRNSNKSK

Nucleotide sequence of GT828 (SEQ ID NO: 15):

ATGCACCATCATCATGAAGGCGTGAGCGACCAGACCCTGAGAGTAACGATGTTT CCGTGGCTTGGGCTGGGTCATGTTAACCCGTTTTTGCGTATCGCTAAACAACTGGCCG ATCGTGGTTTCGTTATCTATTTAGTTAGTACCGCTATTAACCTCGAAATGATCAAAAAG AGAATCCCGGAGAAATACAGTAATAGCATCCATCTGGTTGAGCTGCGCCTGCCAGAAT TACCGGAACTGCCACCACATTACCATACTACCAACGGTTTACCACCGCATCTGAACAA AACCCTGCACAAGGCACTGAAGATGAGCGCTCCCAACTTTAGCAAGATCCTTCAAAA TATTAAGCCGGACCTGGTCCTTTACGATTTTCTGGTTCCGTGGGCAGAAAAAGTCGCG CTTGAACAGGGCATCCCGGCTGTTCCATTGCTAACCAGTGGTGCGGCACTGTTCAGC TACTTTTTCAACTTCCTGAAGCGACCGGGTGAAGAGTTTCCGTTTGAGGCAATCCGCC TGTCGAAGCGAGAACAGGATAAGTTGCGCGAGATGTTGGGAACATTTCCGCCTGAAG AAGATTTTTTAGCGCCGGCCCAGGCCGGTATCATGCTGATGTGCACGAGCCGCGTAAT TGAGGCTAAGTACCTGGACTATTGTACCGAACTGACCAATGTAAAAGTTGTTCCGGTT GGTCCGCCGTTTCAGGATCCGCTGACCGAAGATATTGACGACCCCGAACTGATGGATT GGTTAGATACCAAACCCGAACATAGTGTTGTCTATGTGTCGTTTGGCAGCGAAGCGTT CCTGAGCCGTGAAGATATGGAAGAAGTCGCGTTCGGCCTGGAGCTGAGCGGCGTGA ACTTTATCTGGGTTGCACGCTTTCCGAAAGGCGAAGAACAGCGTCTGGAAGACGTTC TGCCAAAAGGCTTCCTGGAACGCGTTGGTGATCGTGGTCGCGTTCTGAACAAACTGG

TGCCGCAGGCCCATATTCTGAACCATCCGAGCACGGGTGGCTTCATCTCTCATTGCGG
TTGGAACAGCGTCATGGAAAGCATTGATTTCGGCGTTCCGATCATTGCGATGCCGATG
CAGTGGGATCAGCCGATTAACGCGAGACTGCTTGTGGAATTAGGCGTGGCAGTGGAG
ATCCCGCGTGATGAAGATGGCCGGGTCCACCGCGCCGAAATTGCCGAAGCACTGAAA
AGCGTTGTGACCGGTGAAACCGGCGAAATCCTGCGTGCGAAAGTTCGTGAAATCAG
CAAAAACCTGAAATCCATCCGTGACGAAGAAATGGACGCGGTTGCTGAAGAACTGAT
CCAGCTGTGCCGTAACTCTAACAAAAGCAAA

Amino acid sequence of GT828 (SEQ ID NO: 16):

MHHHHEGVSDQTLRVTMFPWLGLGHVNPFLRIAKQLADRGFVIYLVSTAINLEMIK
KRIPEKYSNSIHLVELRLPELPELPPHYHTTNGLPPHLNKTLHKALKMSAPNFSKILQNIKP
DLVLYDFLVPWAEKVALEQGIPAVPLLTSGAALFSYFFNFLKRPGEEFPFEAIRLSKREQDK
LREMLGTFPPEEDFLAPAQAGIMLMCTSRVIEAKYLDYCTELTNVKVVPVGPPFQDPLTE
DIDDPELMDWLDTKPEHSVVYVSFGSEAFLSREDMEEVAFGLELSGVNFIWVARFPKGE
EQRLEDVLPKGFLERVGDRGRVLNKLVPQAHILNHPSTGGFISHCGWNSVMESIDFGVPII
AMPMQWDQPINARLLVELGVAVEIPRDEDGRVHRAEIAEALKSVVTGETGEILRAKVREI
SKNLKSIRDEEMDAVAEELIQLCRNSNKSK

Nucleotide sequence of GT829 (SEQ ID NO: 17):

ATGCACCATCATCATGAAGGCGTGAGCGACCAGACCCTGAGAGTAACGATGTTT
CCGTGGCTTGGGCTGGGTCATGTTAACCCGTTTTTGCGTATCGCTAAACAACTGGCCG
ATCGTGGTTTCGTTATCTATTTAGTTAGTACCGCTATTAACCTCGAAATGATCAAAAAG
AGAATCCCGGAGAAATACAGTAATAGCATCCATCTGGTTGAGCTGCGCCTGCCAGAAT
TACCGGAACTGCCACCACATTACCATACTACCAACGGTTTACCACCGCATCTGAACAA
AACCCTGCACAAGGCACTGAAGATGAGCGCTCCCAACTTTAGCAAGATCCTTCAAAA
TATTAAGCCGGACCTGGTCCTTTACGATTTTCTGGTTCCGTGGGCAGAAAAAGTCGCG
CTTGAACAGGGCATCCCGGCTGTTCCATTGCTAACCAGTGGTGCGGCACTGTTCAGC
TACTTTTTCAACTTCCTGAAGCGACCGGGTGAAGAGTTTCCGTTTGAGGCAATCCGCC
TGTCGAAGCGAGAACAGGATAAGATGCGCGAGATGTTTGGAACAGAGCCGCCTGAA
GAAGATTTTTTAGTTCCGTTTCAGGCCCCTATCATGCTGATGTGCACGAGCCGCGTAAT
TGAGGCTAAGTACCTGGACTATTGTACCGAACTGACCAATGTAAAAGTTGTTCCGGTT
GGTCCGCCGTTTCAGGATCCGCTGACCGAAGATATTGACGACCCCGAACTGATGGATT
GGTTAGATACCAAACCCGAACATAGTGTTGTCTATGTGTCGTTTGGCAGCGAAGCGTT

CCTGAGCCGTGAAGATATGGAAGAAGTCGCGTTCGGCCTGGAGCTGAGCGGCGTGAACTTTATCTGGGTTGCACGCTTTCCGAAAGGCGAAGAACAGCGTCTGGAAGACGTTCTGCCAAAAGGCTTCCTGGAACGCGTTGGTGATCGTGGTCGCGTTCTGAACAAACTGGTGCCGCAGGCCCATATTCTGAACCATCCGAGCACGGGTGGCTTCATCTCTCATTGCGGTTGGAACAGCGTCATGGAAAGCATTGATTTCGGCGTTCCGATCATTGCGATGCCGATGCAGTGGGATCAGCCGATTAACGCGAGACTGCTTGTGGAATTAGGCGTGGCAGTGGAGATCCCGCGTGATGAAGATGGCCGGGTCCACCGCGCCGAAATTGCCGAAGCACTGAAAAGCGTTGTGACCGGTGAAACCGGCGAAATCCTGCGTGCGAAAGTTCGTGAAATCAGCAAAAACCTGAAATCCATCCGTGACGAAGAAATGGACGCGGTTGCTGAAGAACTGATCCAGCTGTGCCGTAACTCTAACAAAAGCAAA

Amino acid sequence of GT829 (SEQ ID NO: 18):

MHHHHEGVSDQTLRVTMFPWLGLGHVNPFLRIAKQLADRGFVIYLVSTAINLEMIKKRIPEKYSNSIHLVELRLPELPELPPHYHTTNGLPPHLNKTLHKALKMSAPNFSKILQNIKPDLVLYDFLVPWAEKVALEQGIPAVPLLTSGAALFSYFFNFLKRPGEEFPFEAIRLSKREQDKMREMFGTEPPEEDFLVPFQAPIMLMCTSRVIEAKYLDYCTELTNVKVVPVGPPFQDPLTEDIDDPELMDWLDTKPEHSVVYVSFGSEAFLSREDMEEVAFGLELSGVNFIWVARFPKGEEQRLEDVLPKGFLERVGDRGRVLNKLVPQAHILNHPSTGGFISHCGWNSVMESIDFGVPIIAMPMQWDQPINARLLVELGVAVEIPRDEDGRVHRAEIAEALKSVVTGETGEILRAKVREISKNLKSIRDEEMDAVAEELIQLCRNSNKSK

Nucleotide sequence of GT830 (SEQ ID NO: 19):

ATGCACCATCATCATGAAGGCGTGAGCGACCAGACCCTGAGAGTAACGATGTTTCCGTGGCTTGGGCTGGGTCATGTTAACCCGTTTTTGCGTATCGCTAAACAACTGGCCGATCGTGGTTTCGTTATCTATTTAGTTAGTACCGCTATTAACCTCGAAATGATCAAAAAGAGAATCCCGGAGAAATACAGTAATAGCATCCATCTGGTTGAGCTGCGCCTGCCAGAATTACCGGAACTGCCACCACATTACCATACTACCAACGGTTTACCACCGCATCTGAACAAAACCCTGCACAAGGCACTGAAGATGAGCGCTCCCAACTTTAGCAAGATCCTTCAAAATATTAAGCCGGACCTGGTCCTTTACGATTTTCTGGTTCCGTGGGCAGAAAAAGTCGCGCTTGAACAGGGCATCCCGGCTGTTCCATTGCTAACCAGTGGTGCGGCACTGTTCAGCTACTTTTTCAACTTCCTGAAGCGACCGGGTGAAGAGTTTCCGTTTGAGGCAATCCGCCTGTCGAAGCGAGAACAGGATAAGATGCGCGAGATGTTTGGAACAGAGCCGCCTGAAGAAGATTTTTTAGCGCCGGCCCAGGCCGGTATCATGCTGATGTGCACGAGCCGCGTAA

TTGAGGCTAAGTACCTGGACTATTGTACCGAACTGACCAATGTAAAAGTTGTTCCGGT
TGGTCCGCCGTTTCAGGATCCGCTGACCGAAGATATTGACGACCCCGAACTGATGGAT
TGGTTAGATACCAAACCCGAACATAGTGTTGTCTATGTGTCGTTTGGCAGCGAAGCGT
TCCTGAGCCGTGAAGATATGGAAGAAGTCGCGTTCGGCCTGGAGCTGAGCGGCGTG
AACTTTATCTGGGTTGCACGCTTTCCGAAAGGCGAAGAACAGCGTCTGGAAGACGTT
CTGCCAAAAGGCTTCCTGGAACGCGTTGGTGATCGTGGTCGCGTTCTGAACAAACTG
GTGCCGCAGGCCCATATTCTGAACCATCCGAGCACGGGTGGCTTCATCTCTCATTGCG
GTTGGAACAGCGTCATGGAAAGCATTGATTTCGGCGTTCCGATCATTGCGATGCCGAT
GCAGTGGGATCAGCCGATTAACGCGAAACTGATGGTGGAATTAGGCGTGGCAGTGGA
GATCCCGCGTGATGAAGATGGCCGGGTCCACCGCGCCGAAATTGCCGAAGCACTGAA
AAGCGTTGTGACCGGTGAAACCGGCGAAATCCTGCGTGCGAAAGTTCGTGAAATCA
GCAAAAACCTGAAATCCATCCGTGACGAAGAAATGGACGCGGTTGCTGAAGAACTG
ATCCAGCTGTGCCGTAACTCTAACAAAAGCAAA

Amino acid sequence of GT830 (SEQ ID NO: 20):

MHHHHEGVSDQTLRVTMFPWLGLGHVNPFLRIAKQLADRGFVIYLVSTAINLEMIK
KRIPEKYSNSIHLVELRLPELPELPPHYHTTNGLPPHLNKTLHKALKMSAPNFSKILQNIKP
DLVLYDFLVPWAEKVALEQGIPAVPLLTSGAALFSYFFNFLKRPGEEFPFEAIRLSKREQDK
MREMFGTEPPEEDFLAPAQAGIMLMCTSRVIEAKYLDYCTELTNVKVVPVGPPFQDPLTE
DIDDPELMDWLDTKPEHSVVYVSFGSEAFLSREDMEEVAFGLELSGVNFIWVARFPKGE
EQRLEDVLPKGFLERVGDRGRVLNKLVPQAHILNHPSTGGFISHCGWNSVMESIDFGVPII
AMPMQWDQPINAKLMVELGVAVEIPRDEDGRVHRAEIAEALKSVVTGETGEILRAKVRE
ISKNLKSIRDEEMDAVAEELIQLCRNSNKSK

Nucleotide sequence of GT831 (SEQ ID NO: 21):

ATGCACCATCATCATGAAGGCGTGAGCGACCAGACCCTGAGAGTAACGATGTTT
CCGTGGCTTGGGCTGGGTCATGTTAACCCGTTTTTGCGTATCGCTAAACAACTGGCCG
ATCGTGGTTTCGTTATCTATTTAGTTAGTACCGCTATTAACCTCGAAATGATCAAAAAG
AGAATCCCGGAGAAATACAGTAATAGCATCCATCTGGTTGAGCTGCGCCTGCCAGAAT
TACCGGAACTGCCACCACATTACCATACTACCAACGGTTTACCACCGCATCTGAACAA
AACCCTGCACAAGGCACTGAAGATGAGCGCTCCCAACTTTAGCAAGATCCTTCAAAA
TATTAAGCCGGACCTGGTCCTTTACGATTTTCTGGTTCCGTGGGCAGAAAAAGTCGCG
CTTGAACAGGGCATCCCGGCTGTTCCATTGCTAACCAGTGGTGCGGCACTGTTCAGC

TACTTTTTCAACTTCCTGAAGCGACCGGGTGAAGAGTTTCCGTTTGAGGCAATCCGCCTGTCGAAGCGAGAACAGGATAAGATGCGCGAGATGTTTGGAACAGAGCCGCCTGAAGAAGATTTTTTAGCGCCGGCCCAGGCCGGTATCATGCTGATGTGCACGAGCCGCGTAATTGAGGCTAAGTACCTGGACTATTGTACCGAACTGACCAATGTAAAAGTTGTTCCGGTTGGTCCGCCGTTTCAGGATCCGCTGACCGAAGATATTGACGACCCCGAACTGATGGATTGGTTAGATACCAAACCCGAACATAGTGTTGTCTATGTGTCGTTTGGCAGCGAAGCGTTCCTGAGCCGTGAAGATATGGAAGAAGTCGCGTTCGGCCTGGAGCTGAGCGGCGTGAACTTTATCTGGGTTGCACGCTTTCCGAAAGGCGAAGAACAGCGTCTGGAAGACGTTCTGCCAAAAGGCTTCCTGGAACGCGTTGGTGATCGTGGTCGCGTTCTGAACAAATTCGCGCCGCAGGCCCATATTCTGAACCATCCGAGCACGGGTGGCTTCATCTCTCATTGCGGTTGGAACAGCGTCATGGAAAGCATTGATTTCGGCGTTCCGATCATTGCGATGCCGATGCAGTGGGATCAGCCGATTAACGCGAGACTGCTTGTGGAATTAGGCGTGGCAGTGGAGATCCCGCGTGATGAAGATGGCCGGGTCCACCGCGCCGAAATTGCCGAAGCACTGAAAAGCGTTGTGACCGGTGAAACCGGCGAAATCCTGCGTGCGAAAGTTCGTGAAATCAGCAAAAACCTGAAATCCATCCGTGACGAAGAAATGGACGCGGTTGCTGAAGAACTGATCCAGCTGTGCCGTAACTCTAACAAAAGCAAA

Amino acid sequence of GT831 (SEQ ID NO: 22):

MHHHHEGVSDQTLRVTMFPWLGLGHVNPFLRIAKQLADRGFVIYLVSTAINLEMIKKRIPEKYSNSIHLVELRLPELPELPPHYHTTNGLPPHLNKTLHKALKMSAPNFSKILQNIKPDLVLYDFLVPWAEKVALEQGIPAVPLLTSGAALFSYFFNFLKRPGEEFPFEAIRLSKREQDKMREMFGTEPPEEDFLAPAQAGIMLMCTSRVIEAKYLDYCTELTNVKVVPVGPPFQDPLTEDIDDPELMDWLDTKPEHSVVYVSFGSEAFLSREDMEEVAFGLELSGVNFIWVARFPKGEEQRLEDVLPKGFLERVGDRGRVLNKFAPQAHILNHPSTGGFISHCGWNSVMESIDFGVPIIAMPMQWDQPINARLLVELGVAVEIPRDEDGRVHRAEIAEALKSVVTGETGEILRAKVREISKNLKSIRDEEMDAVAEELIQLCRNSNKSK

Nucleotide sequence of GT109 (SEQ ID NO: 23):

ATGCCGAACACTAACCCAACTACCGTGCGTCGTCGTCGTGTTATTATGTTTCCGGTTCCGTTCCCGGGCCACTTAAACCCGATGCTGCAACTGGCGAACGTGCTGTACCGTAGAGGTTTTGAAATCACCATTCTGCACACCAACTTCAACGCCCCGAAAACCAGCCTTTATCCGCACTTCCAGTTTCGTTTTATCTTGGACAACGATCCGCAACCGGAGTGGTTACGCAACCTGCCGACGACTGGTCCGGGCGTGGGTGCAAGAATCCCGGTAATTAACAAACA

CGGCGCGGATGAATTCCGTAAGGAGCTGGAAATCTGCATGCGGGATACTCCGAGTGA
CGAGGAAGTTGCTTGCGTGATTACCGATGCGCTGTGGTACTTCGCGCAACCGGTGGC
GGACAGCCTGAATCTGAAACGTCTGGTTCTGCAGACCGGGAGCCTGTTTAACTTCCA
CTGCCTGGTGTGTCTGCCGAAATTTCTGGAGTTGGGCTACCTGGATCCGGAAACTAA
ACATCGTCCGGATGAACCGGTGGTAGGTTTCCCGATGCTGAAGGTTAAAGATATCCGT
CGCGCGTATTCGCACATTCAAGAATCGAAACCAATTCTGATGAAGATGGTTGAAGAA
ACCCGTGCCAGCAGCGGTGTGATTTGGAACAGCGCTAAAGAGCTGGAGGAAAGCGA
GCTGGAAACCATTCAGCGTGAAATTCCGGCGCCGAGCTTCCTGCTTCCGCTGCCGAA
GCATTATAGGGCTTCGAGCACTAGCCTGCTGGATACTGATCCGAGCACCGCCCAATGG
CTGGACCAGCAGCCGCCGAGCAGCGTGCTGTACGTTGGCTTTGGCAGCCAGAGCTC
GCTGGACCCCGCAGATTTCCTGGAGATTGCGCGTGGTCTGGTTGCGAGCAAACAAAG
CTTTCTGTGGAACGTTCGTCCGGGCTTCGTGAAGGGTTATGAGTGGATTGAGCTGCTG
CCGGATGGTTTTCTGGGTGAAAAAGGTCGTATCGTGAAGTCTGCTCCGCAACAAGAA
GTGCTGGCGCACAAGGCGATTGGTGCGTTCTGGACCCACGGCGGTTGGAACGGCAC
CATGGAGGCCGTGTGCGAAGGCGTGCCGATGATCTTTAGCGATTTCGGTCTGGATCAG
CCGCTGAACGCGCGTTACATGAGCGAGGTTCTGCATGTGGGCGTTTATCTGGAGAAC
GGCTTCATCCGTGGTGAGATCATTAATGCGGTTAGGCGTGTGATGGTTGACCCTGAGG
GTGAGGTTATGCGCCAAAACGCGCGTAAATTGAAGGATAAGTTGGATCGAAGCATTG
CTCCCGGTGGCAGCAGCTACGAGAGCCTGGAACGCCTGGAGAGCTATATTAGCAGCC
TG

Amino acid sequence of GT109 (SEQ ID NO: 24):

MPNTNPTTVRRRRVIMFPVPFPGHLNPMLQLANVLYRRGFEITILHTNFNAPKTSLY
PHFQFRFILDNDPQPEWLRNLPTTGPGVGARIPVINKHGADEFRKELEICMRDTPSDEEV
ACVITDALWYFAQPVADSLNLKRLVLQTGSLFNFHCLVCLPKFLELGYLDPETKHRPDEP
VVGFPMLKVKDIRRAYSHIQESKPILMKMVEETRASSGVIWNSAKELEESELETIQREIPA
PSFLLPLPKHYRASSTSLLDTDPSTAQWLDQQPPSSVLYVGFGSQSSLDPADFLEIARGLV
ASKQSFLWNVRPGFVKGYEWIELLPDGFLGEKGRIVKSAPQQEVLAHKAIGAFWTHGG
WNGTMEAVCEGVPMIFSDFGLDQPLNARYMSEVLHVGVYLENGFIRGEIINAVRRVMV
DPEGEVMRQNARKLKDKLDRSIAPGGSSYESLERLESYISS

While specific embodiments of the invention have been described above, it should be understood by those skilled in the art that these are merely illustrative examples. Various changes or modifications may be made to these embodiments without departing from the principles and essence of the present invention. Therefore, the protection scope of the present invention is defined by the appended claims.

## Claims

1. A β-1,2-glucosyltransferase, wherein the β-1,2-glucosyltransferase has the amino acid sequence shown as SEQ ID NO: 4 or a mutated amino acid sequence with at least 99% sequence identity compared to the amino acid sequence shown as SEQ ID NO: 4.

2. The β-1,2-glucosyltransferase of claim 1, wherein, compared to the amino acid sequence shown as SEQ ID NO: 4, the mutated amino acid sequence comprises any four or more of the following amino acid residue differences:

    D323N;
    H324K;
    M181L;
    F185L;
    E188F;
    A196V;
    A198F;
    G201P;
    R373K;
    L375M;
    L325F;
    V326A;
    preferably, the mutated amino acid sequence comprises D323N, H324K, and any two or more of the following amino acid residue differences:

        M181L;
        F185L;
        E188F;
        A196V;
        A198F;
        G201P;
        R373K;
        L375M;
        L325F;
        V326A.

3. The β-1,2-glucosyltransferase of claim 1, wherein the mutated amino acid sequence comprises the following amino acid residue differences compared to the amino acid sequence shown as SEQ ID NO: 4: D323N, H324K, M181L, F185L and E188F; or,

    the mutated amino acid sequence comprises the following amino acid residue differences compared to the amino acid sequence shown as SEQ ID NO: 4: D323N, H324K, A196V, A198F, and G201P; or,
    the mutated amino acid sequence comprises the following amino acid residue differences compared to the amino acid sequence shown as SEQ ID NO: 4: D323N, H324K, R373K, and L375M; or,
    the mutated amino acid sequence comprises the following amino acid residue differences compared to the amino acid sequence shown as SEQ ID NO: 4: D323N, H324K, L325F, and V326A; or,
    the mutated amino acid sequence is shown as SEQ ID NO: 16, SEQ ID NO: 18, SEQ ID NO: 20 or SEQ ID NO: 22.

4. An isolated nucleic acid molecule, wherein the nucleic acid molecule encodes the β-1,2-glucosyltransferase of any one of claims 1-3;
preferably, the nucleotide sequence of the nucleic acid molecule is shown as SEQ ID NO: 3, SEQ ID NO: 15, SEQ ID NO: 17, SEQ ID NO: 19 or SEQ ID NO: 21.

5. A recombinant expression vector, wherein the recombinant expression vector comprises a backbone vector and the isolated nucleic acid molecule of claim 4;

    preferably, the backbone vector comprises a phage vector, a plasmid vector or a viral vector;
    more preferably, the plasmid vector comprises pET28a or pET21a.

6. A transformant, wherein the transformant is a genetically recombinant cell obtained by the introduction of the isolated nucleic acid molecule of claim 4 or the recombinant expression vector of claim 5 into a host cell;

preferably , the host cell is a eukaryotic cell or a prokaryotic cell;
more preferably , the prokaryotic cell is *Escherichia coli,* preferably BL21 (DE3);
further more preferably, the transformant is selected from any one or more of the following strains numbered as RMIS708, RMIS828, RMIS829, RMIS830 and RMIS831 of Abiochem Biotechnology (Shanghai) Co., Ltd.

7. A method for preparing the β-1,2-glucosyltransferase of any one of claims 1-3, wherein the method comprises culturing the transformant of claim 6 in a culture medium to obtain the β-1,2-glucosyltransferase;

preferably, the culture medium is selected from LB liquid medium and/or TB liquid medium; and/or,
the condition for the culturing is: shaking culture at 37 ± 1°C.

8. A preparation method of rebaudioside D, wherein the preparation method comprises the step of combining at least rebaudioside A, a glycosyl donor and the β-1,2-glucosyltransferase of any one of claims 1-3 to form a reaction system and proceeding the reaction.

9. The preparation method of claim 8, wherein the β-1,2-glucosyltransferase is used in the form of wet cells, bacterial powder, liquid enzyme, solid enzyme powder or immobilized enzyme; and/or,

the addition amount of rebaudioside A in the reaction system is 20-120 g/L, preferably 40-110 g/L, more preferably 50-100 g/L; and/or,
the glycosyl donor is NDP-glucose; NDP is preferably selected from any one or more of adenosine diphosphate, uridine diphosphate, cytidine diphosphate, guanosine diphosphate, thymidine diphosphate and inosine diphosphate;
and/or, the pH of the reaction is 5.0-8.0, preferably 6.0;
and/or, the temperature of the reaction is 20-90°C, preferably 60°C;
and/or, the time of the reaction is 10 minutes to 24 hours, preferably 30 minutes.

10. The preparation method of claim 9, wherein the β-1,2-glucosyltransferase is used in the form of wet cells, and the ratio of the added mass of the wet cells to rebaudioside A in the reaction system can be (0.1-0.8): 1, (0.12-0.6): 1 or (0.24-0.3): 1;
preferably,

the added amount of wet cells in the reaction system can be 6-30g/L or 12-24 g/L; and/or,
the β-1,2-glucosyltransferase is used in the form of liquid enzyme; the liquid enzyme is preferably a crude enzyme solution or reaction enzyme solution; the crude enzyme solution is obtained from wet cells expressing the β-1,2-glucosyltransferase; the reaction enzyme solution is obtained by standing the crude enzyme solution at a constant temperature of 80 ± 10°C for a period of time;
further,
the crude enzyme solution is a homogenized enzyme solution obtained by resuspension and homogenization of the wet cells with a solvent, and the ratio of the added mass of the wet cells to the added volume of the solvent is 1g: (5-10) mL; and/or,
the ratio of the added volume of the crude enzyme solution to the added mass of rebaudioside A in the reaction system can be (0.5-5): 1, (0.6-3): 1 or (1.2-1.5): 1 mL/g.

11. An enzyme combination system, wherein the enzyme combination system comprises:

the β-1,2-glucosyltransferase of any one of claims 1-3, which is capable of catalyzing the reaction of rebaudioside A with a glycosyl donor to produce rebaudioside D; and,
β-1,3-glucosyltransferase, which is capable of catalyzing the reaction of rebaudioside D with a glycosyl donor to produce rebaudioside M;
preferably, the enzyme combination system also includes:

a glycosyl donor synthase, which is capable of catalyzing the reaction of a sugar with NDP to produce the glycosyl donor;
more preferably , the sugar is sucrose, the glycosyl donor is NDP-glucose, and the glycosyl donor synthase is

sucrose synthase;

further,

the glycosyl donor synthase is obtained by fermentation cultivation of the first transformant; the first transformant is a genetically recombinant cell obtained by introducing the nucleotide sequence shown as SEQ ID NO: 1 into the host cell, or a genetically recombinant cell capable of expressing the amino acid sequence shown as SEQ ID NO: 2; and/or,

the β-1,2-glucosyltransferase is obtained by fermentation cultivation of the second transformant; the second transformant is a genetically recombinant cell obtained by introducing the isolated nucleic acid molecule of claim 4 or the recombinant expression vector of claim 5 into the host cell; and/or,

the β-1,3-glucosyltransferase is obtained by fermentation cultivation of the third transformant; the third transformant is a genetically recombinant cell obtained by introducing the nucleotide sequence shown as SEQ ID NO: 23 into the host cell, or a genetically recombinant cell capable of expressing the amino acid sequence shown as SEQ ID NO: 24;

further,

the first transformant is the strain numbered as RMIS609 of Abiochem Biotechnology (Shanghai) Co., Ltd.; and/or,

the second transformant is selected from any one or more of the strains numbered as RMIS708, RMIS828, RMIS829, RMIS830, and RMIS831 of Abiochem Biotechnology (Shanghai) Co., Ltd.; and/or,

the third transformant is the strain numbered as RMIS109 of Abiochem Biotechnology (Shanghai) Co., Ltd.

**12.** A preparation method of rebaudioside M, wherein the preparation method comprises:

the step of combining rebaudioside A, a glycosyl donor and the enzyme combination system of claim 11 to form an enzyme catalytic system and proceeding the reaction; or,

the step of combining rebaudioside A, a glycosyl donor and β-1,2-glucosyltransferase to form an enzyme catalytic system and proceeding the reaction, then adding β-1,3-glucosyltransferase and continuing the reaction;

further,

the β-1,2-glucosyltransferase and/or the β-1,3-glucosyltransferase are used in the form of wet cells, bacterial powder, liquid enzyme, solid enzyme powder or immobilized enzyme;

and/or, the added amount of rebaudioside A in the enzyme catalytic system can be 20-120 g/L, 40-110 g/L or 50-100 g/L;

and/or, the glycosyl donor is NDP-glucose; NDP is preferably selected from any one or more of adenosine diphosphate, uridine diphosphate, cytidine diphosphate, guanosine diphosphate, thymidine diphosphate, and inosine diphosphate;

and/or, the pH of the reaction is 5.0-8.0, preferably 6.0;

and/or, the temperature of the reaction is 20-90°C, preferably 60°C;

and/or, the time of the reaction is 10 minutes to 24 hours, preferably 30 minutes to 6 hours;

further,

the ratio of the added mass of the wet cells to rebaudioside A in the enzyme catalytic system can be (0.1-0.8): 1, (0.12-0.6): 1 or (0.24-0.3): 1; or,

the added amount of the wet cells in the enzyme catalytic system can be 6-30g/L or 12-24 g/L; or,

the liquid enzyme is preferably a crude enzyme solution or reaction enzyme solution; the crude enzyme solution is obtained from wet cells expressing the β-1,2-glucosyltransferase and/or the β-1,3-glucosyltransferase; the reaction enzyme solution is obtained by standing the crude enzyme solution at a constant temperature of 80 ± 10°C for a period of time;

further,

the crude enzyme solution is a homogenized enzyme solution obtained by resuspension and homogenization of the wet cells with a solvent, and the ratio of the added mass of the wet cells to the added volume of the solvent is 1g: (5-10) mL;

and/or, the ratio of the added volume of the crude enzyme solution to the added mass of rebaudioside A in the enzyme catalytic system can be (0.5-5): 1 or (0.6-3): 1 or (1.2-1.5): 1 mL/g.

**13.** The preparation method of claim 12, wherein

the wet cells expressing the β-1,2-glucosyltransferase or the liquid enzyme containing the β-1,2-glucosyltransferase is prepared from the second transformant capable of expressing the β-1,2-glucosyltransferase; further, the second transformant is selected from any one or more of the strains numbered as RMIS708, RMIS828, RMIS829, RMIS830 and RMIS831 of Abiochem Biotechnology (Shanghai) Co., Ltd.; and/or,

the wet cells expressing the β-1-3-glucosyltransferase or the liquid enzyme containing the β-1,3-glucosyltransferase is prepared from the third transformant capable of expressing the β-1,3-glucosyltransferase; further, the third transformant is the strain numbered as RMIS109 of Abiochem Biotechnology (Shanghai) Co., Ltd.; and/or, the wet cells expressing the glycosyl donor synthase in the enzyme combination system or the liquid enzyme containing the glycosyl donor synthase is prepared from the first transformant capable of expressing the glycosyl donor synthase; further, the first transformant is the strain numbered as RMIS609 of Abiochem Biotechnology (Shanghai) Co., Ltd.

**14.** The use of the β-1-2-glucosyltransferase of any one of claims 1-3 or the enzyme combination system of claim 11 in preparing rebaudioside D or rebaudioside M with rebaudioside A and a glycosyl donor as substrates.

**15.** An enzyme-catalyzed reaction solution, wherein the enzyme-catalyzed reaction solution comprises: rebaudioside A, glycosyl donor, rebaudioside D and the β-1,2-glucosyltransferase of any one of claims 1-3;
preferably, the enzyme-catalyzed reaction solution also comprises β-1,3-glucosyltransferase and rebaudioside M; and/or, the enzyme-catalyzed reaction solution also comprises: sucrose synthetase, NDP, sucrose, NDP-glucose, and D-fructose.

**16.** An enzyme-catalyzed final product system, **characterized by** comprising:

rebaudioside A;
rebaudioside D;
rebaudioside M;
the β-1,2-glucosyltransferase of any one of claims 1-3; and,
the β-1,3-glucosyltransferase capable of catalyzing the conversion of rebaudioside D and a glycosyl donor to produce the rebaudioside M;
furthermore, in the enzyme-catalyzed final product system, the content of rebaudioside A accounts for less than 1% of the total content of rebaudioside A, rebaudioside D and rebaudioside M; the content of rebaudioside D accounts for less than 9.5% of the total content of rebaudioside A, rebaudioside D and rebaudioside M; and the content of rebaudioside M accounts for more than 84% of the total content of rebaudioside A, rebaudioside D and rebaudioside M.

**17.** A composition, **characterized by** comprising:

rebaudioside A;
rebaudioside D;
rebaudioside M;
wherein, the content of rebaudioside A accounts for less than 1% of the total content of rebaudioside A, rebaudioside D, and rebaudioside M; the content of rebaudioside D accounts for less than 9.5% of the total content of rebaudioside A, rebaudioside D, and rebaudioside M; and the content of rebaudioside M accounts for more than 84% of the total content of rebaudioside A, rebaudioside D, and rebaudioside M.

**18.** A method for producing the target steviol glycoside, wherein the method comprises the following steps:

(a) providing a source of steviol glycoside;
(b) providing a glycosyltransferase;
(c) providing a glycosyl donor or a glycosyl donor generation system;
(d) preparing a reaction mixture using (i) steviol glycoside, (ii) glycosyltransferase, and (iii) glycosyl donor or glycosyl donor generation system, and proceeding the reaction at a temperature of about 30°C to 90°C; wherein steps (a)-(c) above have no specific order;
(e) making the distribution change of the target steviol glycoside reach 50% or more within 2 hours to form a precipitate containing the target steviol glycoside, more preferably, reach 60%, 70%, 80% or 90% or more within 2 hours; optionally, the method also includes:
(f) separating and purifying the precipitate containing the target steviol glycoside from the reaction mixture, the target steviol glycoside is preferably rebaudioside D and/or rebaudioside M.

**19.** The method of claim 18, wherein the source of steviol glycoside is selected from one or more of the following groups: stevia leaf extract, rubusoside, stevioside, rebaudioside A, rebaudioside D.

**20.** The method of claim 19, wherein the steviol glycoside contains at least 60 wt%, 70 wt%, 80 wt%, 90 wt%, 95 wt%, 97 wt%, 98 wt% or more of rebaudioside A.

**21.** The method of claim 18, wherein the glycosyltransferase is selected from one or more of the following groups: β-1,2-glycosyltransferases and β-1,3-glycosyltransferases.

**22.** The method of claim 21, wherein the β-1,2 glycosyltransferase is a glycosyltransferase that performs β-1,2 glycosylation on the C2' position of 13-O-glucose, 19-O-glucose, or 13-O-glucose and 19-O-glucose in steviol glycosides; and the β-1,3 glycosyltransferase is a glycosyltransferase that performs β-1,3 glycosylation on the C3' position of 13-O-glucose, 19-O-glucose, or 13-O-glucose and 19-O-glucose in steviol glycosides.

**23.** The method of claim 21, wherein the glycosyltransferase is thermostable.

**24.** The method of claim 18, wherein the glycosyl donor comprises NDP-sugar.

**25.** The method of claim 24, wherein the NDP-sugar is selected from ADP-glucose, GDP-glucose, CDP-glucose, UDP-glucose, TDP-glucose, IDP-glucose or a combination thereof.

**26.** The method of claim 18, wherein the glycosyl donor generation system comprises sucrose, a glycosyl donor synthase, and NDP.

**27.** The method of claim 26, wherein the glycosyl donor synthase is thermostable.

**28.** The method of claim 26, wherein at the beginning of the reaction, the molar ratio of sucrose to the steviol glycoside in the reaction mixture is 4:1 to 10:1, preferably 4.5:1 to 5:1; and/or the concentration of sucrose is 100 g/L to 200 g/L, preferably 130 g/L to 140 g/L; and/or the concentration of the steviol glycoside is 20 g/L to 120 g/L, preferably 70 g/L to 80 g/L; and/or the reaction temperature is 55°C to 65°C, preferably 60°C; and/or the reaction pH is 5.0 to 8.0, preferably 6.0 to 6.2.

**29.** The method of claim 28, wherein the pH is reached by adding food-grade acid.

**30.** The method of claim 29, wherein the food-grade acid is selected from the following groups: phosphoric acid, acetic acid and citric acid.

**31.** The method of claim 21, wherein the amino acid sequence of the β-1,3-glycosyltransferase has at least about 90% identity with SEQ ID NO: 24; and/or the β-1,2-glycosyltransferase is the β-1,2-glucosyltransferase of any of claims 1-3.

**32.** The method of claim 31, wherein the nucleotide sequence of the gene encoding the β-1,3-glycosyltransferase has at least about 90% identity with SEQ ID NO: 23; and/or the gene encoding the β-1,2 glycosyltransferase is the nucleic acid molecule of claim 4.

**33.** The method of claim 26, wherein the amino acid sequence of the glycosyl donor synthase has at least about 90% identity with SEQ ID NO: 2.

**34.** The method of claim 33, wherein the nucleotide sequence of the gene encoding the glycosyl donor synthase has at least 90% identity with SEQ ID NO: 1.

**35.** The method of claim 18, wherein the distribution change of the steviol glycoside reaches 85% or more within 30 minutes to 4 hours.

**36.** A consumable comprising rebaudioside D and/or rebaudioside M, prepared by the method of any one of claims 18-35.

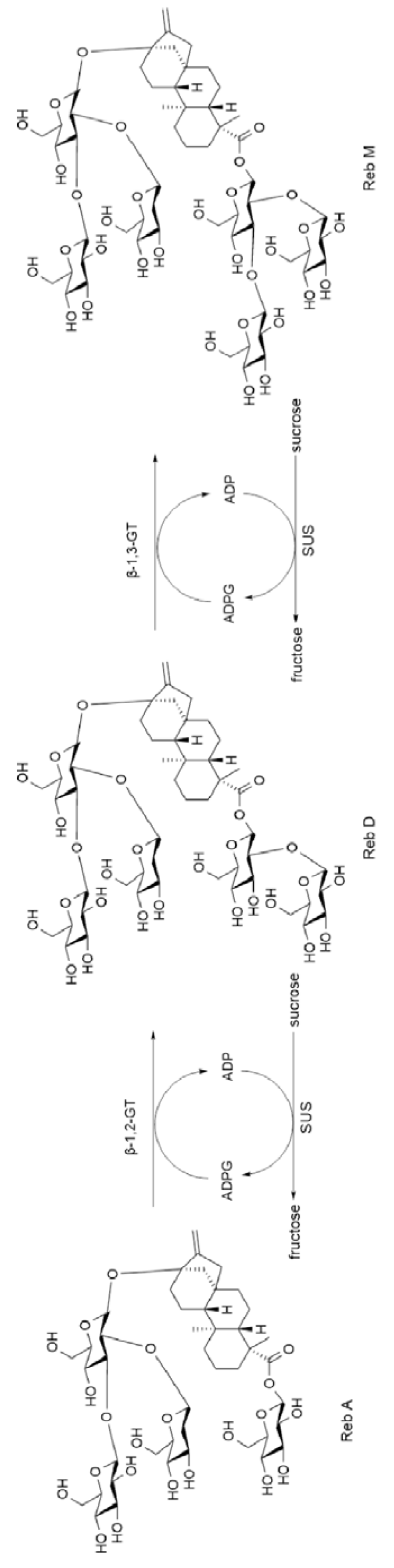
Reb A
β-1,2-GT
ADPG
ADP
SUS
fructose
sucrose
Reb D
β-1,3-GT
ADPG
ADP
SUS
fructose
sucrose
Reb M

FIG. 1

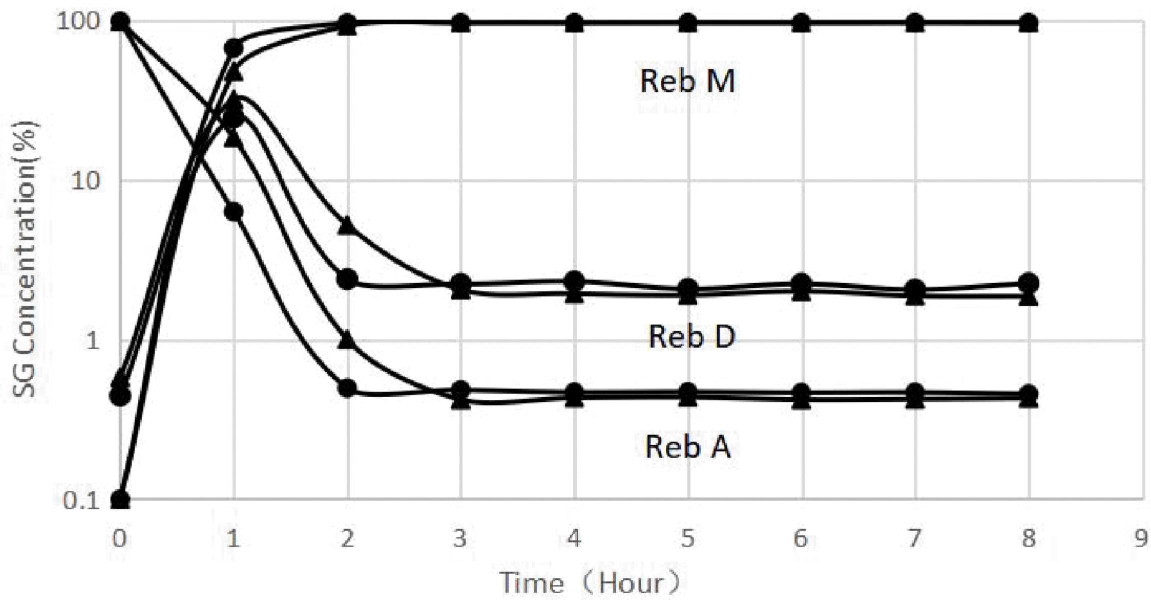
100
10
1
0.1
SG Concentration(%)
Reb M
Reb D
Reb A
0
1
2
3
4
5
6
7
8
9
Time（Hour）

FIG. 2

**INTERNATIONAL SEARCH REPORT**

International application No.
**PCT/CN2024/114579**

**A. CLASSIFICATION OF SUBJECT MATTER**

C12N9/10(2006.01)i; C12N15/63(2006.01)i; C12P19/00(2006.01)i

According to International Patent Classification (IPC) or to both national classification and IPC

**B. FIELDS SEARCHED**

Minimum documentation searched (classification system followed by classification symbols)

IPC: C12N, C12P

Documentation searched other than minimum documentation to the extent that such documents are included in the fields searched

Electronic data base consulted during the international search (name of data base and, where practicable, search terms used)

VEN, CNABS, PubMed, ISI Web of Science, VCN, CNKI, 万方, WANFANG, 百度学术, BAIDU SCHOLAR, 读秀, DUXIU, STN, GenBank, 中国专利生物序列检索系统, China Patents Biological Sequence Search System: β-1, 2-葡萄糖基转移酶, glucosyltransferase, mutant, D323N, H324K, M181L, F185L, E188F, A196V, A198F, G201P, R373K, L375M, L325F, V326A, SEQ ID NOs: 4, 16, 18, 20, 22, 莱鲍迪苷, rebaudioside, 弈柯莱生物科技, 吴燕, WU, Y.

**C. DOCUMENTS CONSIDERED TO BE RELEVANT**

| Category* | Citation of document, with indication, where appropriate, of the relevant passages | Relevant to claim No. |
|---|---|---|
| X | WO 2022257983 A1 (ABIOCHEM BIOTECHNOLOGY (SHANGHAI) CO., LTD.) 15 December 2022 (2022-12-15)<br>claims 1-15, sequence table, sequence 2, and description, paragraphs 41-68 | 17-36 |
| A | WO 2022257983 A1 (ABIOCHEM BIOTECHNOLOGY (SHANGHAI) CO., LTD.) 15 December 2022 (2022-12-15)<br>claims 1-15, sequence table, sequence 2, and description, paragraphs 41-68 | 1-16 |
| A | CN 112805295 A (CODEXIS, INC. et al.) 14 May 2021 (2021-05-14)<br>claims 1-131, and sequence table, sequence 2 | 1-36 |
| A | CN 107075489 A (NOVOZYMES A/S) 18 August 2017 (2017-08-18)<br>claims 1-32 | 1-36 |
| A | CN 113462670 A (NANJING TECH UNIVERSITY) 01 October 2021 (2021-10-01)<br>claims 1-9 | 1-36 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 November 2024** | **15 November 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No. **PCT/CN2024/114579**

**C. DOCUMENTS CONSIDERED TO BE RELEVANT**

| Category* | Citation of document, with indication, where appropriate, of the relevant passages | Relevant to claim No. |
|---|---|---|
| A | 陈红 等 (CHEN, Hong et al.). "一个潜在催化莱鲍迪D苷合成的新型糖基转移酶 (A Novel Glycosyltransferase Potentially Catalyzing the Synthesis of Rebaudioside D)" *分子植物育种 (Molecular Plant Breeding),* Vol. 18, No. 09, 31 December 2020 (2020-12-31), 2872-2877<br>abstract | 1-36 |
| A | CIOCCHINI, A. E. et al. "A glycosyltransferase with a length-controlling activity as a mechanism to regulate the size of polysaccharides" *PNAS,* Vol. 104, No. 42, 16 October 2007 (2007-10-16), 16492-16497<br>abstract | 1-36 |
| A | KOCINCOVA, D. et al. "Evidence that WapB Is a 1,2-Glucosyltransferase of Pseudomonas aeruginosa Involved in Lipopolysaccharide Outer Core Biosynthesis" *JOURNAL OF BACTERIOLOGY,* Vol. 193, No. 11, 30 June 2011 (2011-06-30), 2708–2716<br>abstract | 1-36 |

Form PCT/ISA/210 (second sheet) (July 2022)

**INTERNATIONAL SEARCH REPORT**

International application No.
**PCT/CN2024/114579**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/114579**

| Patent document cited in search report | Publication date (day/month/year) | Patent family member(s) | Publication date (day/month/year) |
|---|---|---|---|
| WO 2022257983 A1 | 15 December 2022 | None | |
| CN 112805295 A | 14 May 2021 | CA 3108268 A1 | 06 February 2020 |
| | | SG 11202100470 WA | 25 February 2021 |
| | | MX 2021001260 A | 12 April 2021 |
| | | JP 2021532757 A | 02 December 2021 |
| | | JP 2022132485 A | 08 September 2022 |
| | | WO 2020028039 A1 | 06 February 2020 |
| | | KR 20210040408 A | 13 April 2021 |
| | | BR 112021001661 A2 | 04 May 2021 |
| | | BR 112021001661 A8 | 09 August 2022 |
| | | US 2020032227 A1 | 30 January 2020 |
| | | US 11760981 B2 | 19 September 2023 |
| | | AU 2019314181 A1 | 04 February 2021 |
| | | AU 2019314181 B2 | 17 October 2024 |
| | | TW 202019951 A | 01 June 2020 |
| | | EP 3830106 A1 | 09 June 2021 |
| | | EP 3830106 A4 | 06 July 2022 |
| | | JP 2023039908 A | 22 March 2023 |
| | | US 2024010999 A1 | 11 January 2024 |
| | | IL 280302 A | 01 March 2021 |
| | | JP 2024114921 A | 23 August 2024 |
| CN 107075489 A | 18 August 2017 | WO 2016079305 A1 | 26 May 2016 |
| | | US 2017321199 A1 | 09 November 2017 |
| | | US 10287562 B2 | 14 May 2019 |
| | | EP 3221447 A1 | 27 September 2017 |
| CN 113462670 A | 01 October 2021 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023110745202
- CN 106795523 A
- CN 110914445 A
- CN 113186141 B
- CN 115678867 A
- CN 115418358 A


### Non-patent literature cited in the description


- **OHTA et al.** *J. Appl. Glycosci.*, 2010, vol. 57, 199-209
- **INDRA PRAKASH et al.** *Molecules*, 2014, vol. 19, 17345-17355